# EUROPEAN PATENT SPECIFICATION

(11) **EP 0 652 868 B1**
(45) Date of publication and mention of the grant of the patent: **10.11.2004**
(21) Application number: 93917937.0
(22) Date of filing: 28.07.1993
(51) Int. Cl.: C07D 213/75, C07D 213/89, C07D 213/50, C07C 49/84, C07C 233/67, C07C 275/34, C07C 69/92, C07C 43/243, A61P 29/00

(54) **INHIBITORS OF c-AMP PHOSPHODIESTERASE**
INHIBITOREN VON C-AMP PHOSPHODIESTERASE
INHIBITEURS DE PHOSPHODIESTERASE D'AMP CYCLIQUE

(30) Priority: 28.07.1992 GB 9215989; 28.07.1992 GB 9216005; 28.07.1992 GB 9216006; 28.07.1992 GB 9216008; 07.08.1992 GB 9216764; 21.05.1993 GB 9310633; 27.05.1993 GB 9310938; 01.06.1993 GB 9311281; 16.07.1993 GB 9314847
(43) Date of publication of application: 17.05.1995
(73) Proprietor: Aventis Pharma Limited, West Malling, Kent ME19 4AH (GB)
(72) Inventor: FENTON, Garry, Dagenham, Essex RM10 7XS (GB); MORLEY, Andrew David, Dagenham, Essex RM10 7XS (GB); PALFREYMAN, Malcolm Norman, Dagenham, Essex RM10 7XS (GB); RATCLIFFE, Andrew James, Dagenham, Essex RM10 7XS (GB); SHARP, Brian William, Dagenham, Essex RM10 7XS (GB); STUTTLE, Keith Alfred, James, Dagenham, Essex RM10 7XS (GB); THURAIRATNAM, Sukanthini, Dagenham, Essex RM10 7XS (GB); VACHER, Bernard Yvon Jack, Dagenham, Essex RM10 7XS (GB)
(74) Representative: Jones, Stephen Anthony
(86) International application number: PCT/GB1993/001597
(87) International publication number: WO 1994/002465

(56) References cited:
- EP-A- 0 470 805
- EP-A- 0 497 564
- WO-A-91/15451
- WO-A-92/00968
- WO-A-92/07567
- CHEMICAL ABSTRACTS, vol. 99, no. 6, 8 August 1983, Columbus, Ohio, US; abstract no. 43558, 'Hypoglycemic pharmaceuticals containing benzamide derivatives' page 322 ; cited in the application

## Description

### Field of the Invention

This invention is directed to substituted phenyl compounds, their preparation, pharmaceutical compositions containing these compounds, and their use for the manufacture of a medicament for the treatment of disease states associated with proteins that mediate cellular activity.

Disease states associated with abnormally high physiological levels of cytokines such as TNF are treatable according to the invention. TNF is an important pro-inflammatory cytokine which causes hemorrhagic necrosis of tumors and possesses other important biological activities. TNF is released by activated macrophages, activated T-lymphocytes, natural killer cells, mast cells and basophils, fibroblasts, endothelial cells and brain astrocytes among other cells.

The principal in vivo actions of TNF can be broadly classified as inflammatory and catabolic. It has been implicated as a mediator of endotoxic shock, inflammation of joints and of the airways, immune deficiency states. allograft rejection, and in the cachexia associated with malignant disease and some parasitic infections. In view of the association of high serum levels of TNF with poor prognosis in sepsis, graft versus host disease and acute respiratory distress syndrome, and its role in many other immunologic processes, this factor is regarded as an important mediator of general inflammation.

TNF primes or activates neutrophils, eosinophils, fibroblasts and endothelial cells to release tissue damaging mediators. TNF also activates monocytes, macrophages and T-lymphocytes to cause the production of colony colony stimulating factors and other pro-inflammatory cytokines such IL₁, IL₆, IL₈ and GM-CSF, which in some case mediate the end effects of TNF. The ability of TNF to activate T-lymphocytes, monocytes, macrophages and related cells has been implicated in the progression of Human Immunodeficiency Virus (HIV) infection. In order for these cells to become infected with HIV and for HIV replication to take place the cells must be maintained in an activated state. Cytokines such as TNF have been shown to activate HIV replication in monocytes and macrophages. Features of endotoxic shock such as fever, metabolic acidosis, hypotension and intravascular coagulation are thought to be mediated through the actions of TNF on the hypothalamus and in reducing the anti-coagulant activity of vascular endothelial cells. The cachexia associated with certain disease states is mediated through indirect effects on protein catabolism. TNF also promotes bone resorption and acute phase protein synthesis.

The discussion herein related to disease states associated with TNF include those disease states related to the production of TNF itself, and disease states associated with other cytokines, such as but not limited to IL-1, or IL-6, that are modulated by associated with TNF. For example, a IL-1 associated disease state, where IL-1 production or action is exacerbated or secreted in response to TNF, would therefore be considered a disease state associated with TNF. TNF-α and TNF-β are also herein referred to collectively as "TNF" unless specifically delineated otherwise, since theRE is a close structural homology between TNF-α (cachectin) and TNF-β (lymphotoxin) and each of them has a capacity to induce similar biologic responses and bind to the same cellular receptor.

Disease states associated with pathological conditions that are modulated by inhibiting enzymes, which are associated with secondary cellular messengers, such as cyclic AMP phosphodiesterase are also treatable according to the invention cyclic AMP phosphodiesterase is an important enzyme which regulates cyclic AMP levels and in turn thereby regulates other important biological reactions. The ability to regulate cyclic AMP phosphodiesterase, including type IV cyclic AMP phosphodiesterase, therefore, has been implicated as being capable of treating assorted biological conditions.

In particular, inhibitors of type IV cyclic AMP phosphodiesterase have been implicated as being bronchodilators and asthma-prophylactic agents and as agents for inhibiting eosinophil accumulation and of the function of eosinophils, and for treating other diseases and conditions characterized by, or having an etiology involving, morbid eosinophil accumulation. Inhibitors of cyclic AMP phosphodiesterase are also implicated in treating inflammatory diseases, proliferative skin diseases and conditions associated with cerebral metabolic inhibition.

### Reported Developments

Chemical Abstracts, 108(15), April 11, 1988, abstract no. 131583p pertains to an abstract of Japanese Patent Application Publication No. JP-A-62 158,253 which discloses that a substituted phenyl compound of formula 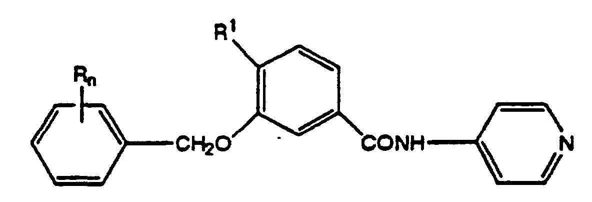 is a cardiotonic, but does not disclose or suggest that the compound inhibits cyclic AMP phosphodiesterase or TNF. JP-A-62 158,253 also does not disclose or suggest that the moiety that is ortho to R¹ may be anything other than benzyloxy.

Chemical Abstracts, 99(6), August 8, 1983, abstract no. 43556z pertains to an abstract of Japanese Patent Application Publication No. JP-A-5 869,812 which discloses that a phenyl compound of formula 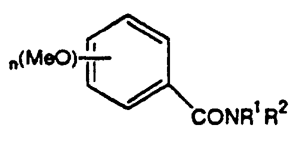 is a hypoglycemic agent, but does not disclose or suggest that the compound inhibits cyclic AMP phosphodiesterase or TNF. JP-A-5 869,812 also does not disclose or suggest that the benzamide moiety may be substituted by anything other than methoxy.

Panos Grammaticakis, Bull. Soc. Chim. Fr, 848-857 (1965) discloses a phenyl compound of the formula 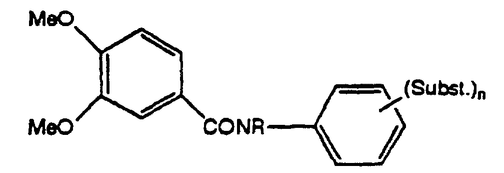 Grammaticakis examines the ultraviolet and visible absorbances of compounds bearing different substituents. Grammaticakis does not disclose or suggest that the compound exhibits any pharmacological activity.

lan W. Mathison, et al., J. Med. Chem., 16(4), 332-336 (1973), discloses that a phenyl compound of formula 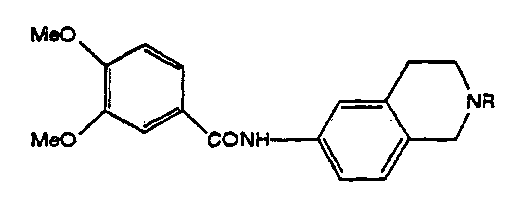 is a hypotensive agent, but do not disclose or suggest that the compound inhibits cyclic AMP phosphodiesterase or TNF. Mathison, et al., also do not disclose or suggest that the benzamide moiety may be substituted by anything other than methoxy.

European Patent Application Publication No. EP 232199 B1 discloses that phenyl compounds of formula 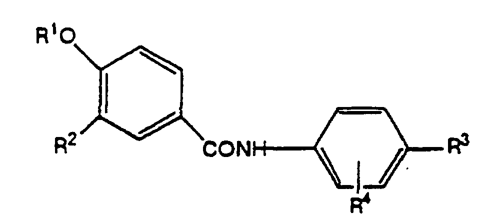 wherein R² is alkyl or mono- or polycyclic cycloalkyl, exhibit anti-inflammatory and/or anti-allergic activity. EP 232199 B1 does not disclose or suggest compounds wherein the R² substituent is bonded to the phenyl moiety via an oxygen or sulfur atom.

European Patent Application Publication No. EP 470,805 A1 discloses phenyl compounds of the formula 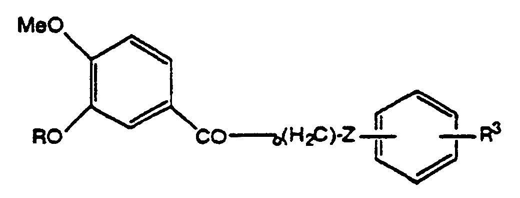 wherein R may be C₃₋₇ alkyl, C₃₋₇ cycloalkyl or 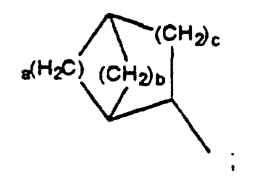 Z may be a bond; o is 1-4; a and b are independently 1-3; and c is 0-2. EP 470,805 A1 discloses that these compounds are useful intermediates for preparing PDE IV inhibitors, but does not disclose or suggest that the compounds have any pharmacological activity.

Japanese Patent Application Publication No. JP-A-0 4360847 discloses compounds of the formula 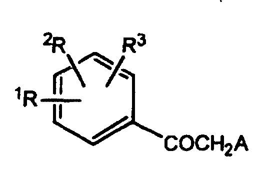 wherein R¹, R² and R³ may be the same or different and may be optionally substituted lower alkyl(O); and A may be optionally substituted aryl or 5-6 membered heterocyclyl group. JP-A-0 4360847 discloses that the compounds areuseful intermediates for preparing antimicrobial agents, but does not disclose or suggest that the compounds have any pharmacological activity.

WO Patent Application No. 92/12961 discloses that compounds of the formula 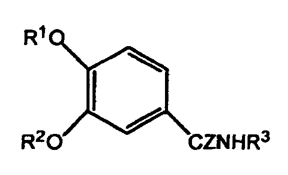 inhibit cyclic AMP phosphodiesterase. WO Patent Application No. 92/12961 does not disclose or suggest that these compound inhibit TNF. WO Patent Application No. 92/12961 also does not disclose compounds wherein R¹ is lower alkyl substituted by halo; or R² is alkyl substituted by halo, cycloalkyl or cycloalkenyl, alkenyl, cycloalkyl substituted by halo, methylene or alkyl, cycloalkenyl, cyclothioalkyl or cyclothioalkenyl; or R¹ or R² attached to the phenyl through sulfur; or R³ is aryl or heteroaryl each of which is substituted by aralkoxy, aralkylthio, carboxy, aralkyloxycarbonyl, Y¹Y²N-, Y¹Y²NCO- or Y¹Y²NSO₂- where Y¹ and Y² are independently hydrogen, alkyl, aryl or aralkyl provided that one or both of Y¹ and Y² is aryl or aralkyl.

EP 0470805 discloses aryl oxime-carbamates and oxime-carbonates and EP 0496564 discloses benzamide derivatives which are cyclic AMP phosphodiesterase inhibitors and are useful as bronchodilators and anti-inflammatory agents.

### SUMMARY OF THE INVENTION

This invention is directed to a pharmaceutical composition as defined in Claim 1, and compounds of formula I as defined in Claim 2.

Compounds within the scope of the present invention also inhibit cyclic AMP phosphodiesterase, and are useful in treating a disease state associated with pathological conditions that are modulated by inhibiting cyclic AMP phosphodiesterase, such disease states including inflammatory and autoimmune diseases, in particular type IV cyclic AMP phosphodiesterase.

### DETAILED DESCRIPTION OF THE INVENTION

As used above, and throughout the description of the invention, the following terms, unless otherwise indicated, shall be understood to have the following meanings:

### Definitions

"Patient" includes both human and other mammals.

"Alkyl" means an aliphatic hydrocarbon group which may be straight or branched having about 1 to about 15 carbon atoms in the chain. Preferred alkyl groups have 1 to about 12 carbon atoms in the chain. Branched means that one or more lower alkyl groups such as methyl, ethyl or propyl are attached to a linear alkyl chain. "Lower alkyl" means about 1 to about 4 carbon atoms in the chain which may be straight or branched. The alkyl group may be substituted by one or more halo, cycloalkyl or cycloalkenyl. Exemplary alkyl groups include methyl, fluoromethyl, difluoromethyl, trifluoromethyl, cyclopropylmethyl, cyclopentylmethyl, ethyl, *n*-propyl, *i*-propyl, *n*-butyl, *t*-butyl, *n*-pentyl, 3-pentyl, heptyl, octyl, nonyl, decyl and dodecyl.

"Alkenyl" means an aliphatic hydrocarbon group containing a carbon-carbon double bond and which may be straight or branched having about 2 to about 15 carbon atoms in the chain. Preferred alkenyl groups have 2 to about 12 carbon atoms in the chain; and more preferably about 2 to about 4 carbon atoms in the chain. Branched means that one or more lower alkyl groups such as methyl, ethyl or propyl are attached to a linear alkenyl chain. "Lower alkenyl" means about 2 to about 4 carbon atoms in the chain which may be straight or branched. The alkenyl group may be substituted by one or more halo. Exemplary alkenyl groups include ethenyl, propenyl, *n*-butenyl, *i*-butenyl, 3-methylbut-2-enyl, *n*-pentenyl, heptenyl, octenyl and decenyl.

"Cycloalkyl" means a non-aromatic mono- or multicyclic ring system of about 3 to about 10 carbon atoms. Preferred monocyclic cycloalkyl rings include cyclopentyl, fluorocyclopentyl, cyclohexyl and cycloheptyl; more preferred is cyclopentyl. The cycloalkyl group may be substituted by one or more halo, methylene (H₂C=) or alkyl. Exemplary multicyclic cycloalkyl rings include 1-decalin, adamant-(1- or 2-)yl and norbomyl.

"Cycloalkenyl" means a non-aromatic monocyclic or multicyclic ring system containing a carbon-carbon double bond and having about 3 to about 10 carbon atoms. Preferred monocyclic cycloalkenyl rings include cyclopentenyl, cyclohexenyl or cycloheptenyl; more preferred is cyclopentenyl. A preferred multicyclic cycloalkenyl ring is norbornylenyl. The cycloalkenyl group may be substituted by one or more halo.

"Cyclothioalkyl" means a non-aromatic monocyclic or multicyclic ring system of about 3 to about 10 ring atoms. Preferred rings include about 5 to about 6 ring atoms wherein one of the ring atoms is sulfur. The cyclothioalkyl may be optionally substituted by one or more halo. Preferred monocyclic cyclothioalkyl rings include tetrahydrothiophenyl and tetrahydrothiopyranyl; more preferred is tetrahydrothiophenyl. The thio moiety of the cyclothioalkyl may also be optionally oxidized to the corresponding S-oxide or S,S-dioxide.

"Cyclothioalkenyl" means a non-aromatic monocyclic or multicyclic ring system containing a carbon-carbon double bond and having about 3 to about 10 ring atoms. Preferred rings include about 5 to about 6 ring atoms and wherein one of the ring atoms is sulfur. The cyclothioalkenyl may be optionally substituted by one or more halo. Preferred monocyclic cyclothioalkyl rings include dihydrothiophenyl and dihydrothiopyranyl; more preferred is dihydrothiophenyl. The thio moiety of the cyclothioalkyl may also be optionally oxidized to the corresponding S-oxide or S,S-dioxide

"Aryl" means aromatic carbocyclic radical containing about 6 to about 10 carbon atoms. Exemplary aryl include phenyl or naphthyl, or phenyl or naphthyl substituted with one or more aryl group substituents which may be the same or different, where "aryl group substituent" includes hydrogen, alkyl, aryl, aralkyl, hydroxy, hydroxyalkyl, alkoxy, aryloxy, aralkoxy, carboxy, acyl, aroyl, halo, nitro, cyano, carboxy, alkoxycarbonyl, aryloxycarbonyl, aralkoxycarbonyl, acylamino, aroylamino, alkylsulfonyl, arylsulfonyl, alkylsulfinyl, arylsulfinyl, alkylthio, arylthio, aralkylthio, Y¹Y²N-, Y¹Y²NCO- or Y¹Y²NSO₂-, where Y¹ and Y² are independently hydrogen, alkyl, aryl, and aralkyl. Preferred aryl group substituents include hydrogen, alkyl, hydroxy, acyl, aroyl, halo, nitro, cyano, alkoxycarbonyl, acylamino, alkylthio, Y¹Y²N-, Y¹Y²NCO- or Y¹Y²NSO₂-, where Y¹ and Y² are independently hydrogen and alkyl.

"Heteroaryl" means about a 5- to about a 10- membered aromatic monocyclic or multicyclic hydrocarbon ring system in which one or more of the carbon atoms in the ring system is/are element(s) other than carbon, for example nitrogen, oxygen or sulfur. The "heteroaryl" may also be substituted by one or more aryl group substituents. Exemplary heteroaryl groups include pyrazinyl, furanyl, thienyl, pyridyl, pyrimidinyl, isoxazolyl, isothiazolyl, quinolinyl, and isoquinolinyl. Preferred heteroaryl groups include pyrazinyl, thienyl, pyridyl, pyrimidinyl, isoxazolyl and isothiazolyl.

"Aralkyl" means an aryl-alkyl- group in which the aryl and alkyl are as previously described. Preferred aralkyls contain a lower alkyl moiety. Exemplary aralkyl groups include benzyl, 2-phenethyl and naphthlenemethyl.

"Hydroxyalkyl" means a HO-alkyl- group in which alkyl is as previously defined. Preferred hydroxyalkyls contain lower alkyl. Exemplary hydroxyalkyl groups include hydroxymethyl and 2-hydroxyethyl.

"Acyl" means an H-CO- or alkyl-CO- group in which the alkyl group is as previously described. Preferred acyls contain a lower alkyl. Exemplary acyl groups include formyl, acetyl, propanoyl, 2-methylpropanoyl, butanoyl and palmitoyl.

"Aroyl" means an aryl-CO- group in which the alkyl group is as previously described. Exemplary groups include benzoyl and 1- and 2-naphthoyl.

"Alkoxy" means an alkyl-O- group in which the alkyl group is as previously described. Exemplary alkoxy groups include methoxy, ethoxy, *n*-propoxy, *i*-propoxy, *n*-butoxy and heptoxy.

"Aryloxy" means an aryl-O- group in which the aryl group is as previously described. Exemplary aryloxy groups include phenoxy and naphthoxy.

"Aralkyloxy" means an aralkyl-O- group in which the aralkyl groups is as previously described. Exemplary aralkyloxy groups include benzyloxy and 1- or 2-naphthalenemethoxy.

"Alkylthio" means an alkyl-S- group in which the alkyl group is as previously described. Exemplary alkylthio groups include methylthio, ethylthio, *i*-propylthio and heptylthio.

"Arylthio" means an aryl-S- group in which the aryl group is as previously described. Exemplary arylthio groups include phenylthio and naphthylthio.

"Aralkylthio" means an aralkyl-S- group in which the aralkyl group is as previously described. An exemplary aralkylthio group is benzylthio.

"Y¹Y²N-" means a substituted or unsubstituted amino group, wherein Y¹ and Y² are as previously described. Exemplary groups include amino (H₂N-), methylamino, ethylmethylamino, dimethylamino and diethylamino.

"Alkoxycarbonyl" means an alkyl-O-CO- group. Exemplary alkoxycarbonyl groups include methoxy- and ethoxycarbonyl.

"Aryloxycarbonyl" means an aryl-O-CO- group. Exemplary aryloxycarbonyl groups include phenoxy- and naphthoxycarbonyl.

"Aralkoxycarbonyl" means an aralkyl-O-CO- group. An exemplary aralkoxycarbonyl group is benzyloxycarbonyl.

"Y¹Y²NCO-" means a substituted or unsubstituted carbamoyl group, wherein Y¹ and Y² are as previously described. Exemplary groups are carbamoyl (H₂NCO-) and dimethylcarbamoyl (Me₂NCO-).

"Y¹Y²NSO₂-" means a substituted or unsubstituted sulfamoyl group, wherein Y¹ and Y² are as previously described. Exemplary groups are sulfamoyl (H₂NSO₂-) and dimethylsulfamoyl (Me₂NSO₂-).

"Acylamino" is an acyl-NH- group wherein acyl is as defined herein.

"Aroylamino" is an aroyl-NH- group wherein aroyl is as defined herein.

"Alkylsulfonyl" means an alkyl-SO₂- group. Preferred groups are those in which the alkyl group is lower alkyl.

"Alkylsulfinyl" means an alkyl-SO- group. Preferred groups are those in which the alkyl group is lower alkyl.

"Arylsulfonyl" means an aryl-SO₂- group.

"Arylsulfinyl" means an aryl-SO- group.

"Halo" means fluoro, chloro, bromo, or iodo. Preferred are fluoro, chloro or bromo, and more preferred are fluoro or chloro.

### Preferred Embodiments

A compound of formula I is preferred for use in the manufacture of a medicament for treating a disease state associated with a physiologically detrimental excess of tumor necrosis factor.

Disease states associated with pathological conditions that are modulated by inhibiting cyclic AMP phosphodiesterase can be treated with a compound of formula I.

Special embodiments of the compounds of the present invention include those wherein R² is norbomyl, norbomenyl, cyclopentyl and cyclopentenyl; preferably cyclopentyl, norbomyl and norbornenyl.

According to a further aspect of the invention, preferred compounds of formula I are described wherein Z¹ and Z² are oxygen, and Z¹ is sulfur and Z² is oxygen are preferred. More preferred are where Z¹ and Z² are oxygen.

Compounds of the invention wherein R¹ is substituted by halo, preferably fluoro, are preferred. It is also preferred that the halo substitution is on positions of the R¹ that are adjacent to the position of R¹ that is attached respectively to Z¹.

Among the compounds of the invention where R³ is substituted phenyl, the phenyl group is preferably substituted on the 2-position or on both the 2- and 6-positions.

Similarly, among compounds of the invention where R³ is substituted heteroaryl, the heteroaryl group is preferably substituted on one or both, more preferably on both, of the positions adjacent to the position of R³ that is attached to Z³. Further preferred are compounds wherein R³ is a 3,5-dihalopyrid-4-yl moiety or an N-oxide thereof.

Preferred compounds for use according to the invention are selected from the following:
- CJ: 3-cyclopentyloxy-4-methoxyphenyl 2',6'-dichlorobenzyl ketone;
- CK: 3-cyclopentyloxy-4-methoxyphenyl 3,5-dichloropyrid-4-ylmethyl ketone;
- CL: 3,5-dichloro-4-(2-(3-cyclopentyloxy-4-methoxyphenyl)-2-oxoethyl)pyridine-N-oxide;
- CM: 1-(3-cyclopentyloxy-4-methoxyphenyl)-2-(3-chtoropyrid-4-yl)ethanone;
- CN: 1-(3-cyclopentyloxy-4-methoxyphenyl)-2-(4-pyridyl)ethanone;
- CR: N-(3-cyclopentyloxy-4-methoxyphenyl)-2,6-dichlorobenzamide;
- CS: N-(3-cyclopentyloxy-4-methoxyphenyl)-2,6-difluorobenzamide;
- CV: (3-cyclopentyloxy-4-methoxyphenyl) 2,6-dichlorobenzoate;
- CW: 3-cyclopentyloxy-4-methoxyphenyl-2,6-dichlorobenzyl ether;
- CX: N-(2-chlorophenyl)-3-cyclopentyloxy-4-methoxybenzylamine;
- CY: 1-(3-cyclopentyloxy-4-methoxyphenyl)-2-(2,6-dichlorophenyl)ethene;
- DA: 1-(3-cyclopentyloxy-4-methoxyphenyl)-2-(pyrid-4-yl)ethane-1,2-dione;
- DB: trans-1 -(3-cyclopentyloxy-4-methoxyphanyl)2-(3,5-dichloropyrid-4-yl)diazene;
- DC: 1-(3-cyclopentyloxy-4-methoxyphenyl)-c-1-oxo-r-2-(3,5-dichloro-1-oxo-pyrid-4-yl)diazene;
- DD: trans-1-(3-cyclopentyloxy-4-methoxyphenyl)-2-(3,5-dichloro-1-oxo-pyrid-4-yl)diazene;
- EC: (±)-1-[3-{(exo)-8,9,10-trinorbomyl-2-oxy}-4-methoxyphenyl]-2-(3,5-dichloropyrid-4-yl)ethanone;
- ED: 1-[3-cyclopentyloxy-4-(methylthio)phenyl]-2-(3,5-dichloropyrid-4-yl)ethanone;
- EE: 1-(4-methoxy-3-prop-2-yloxyphenyl)-2-(3,5-dichloropyrid-4-yl)ethanone;
- EF: 1-(4-methylthio-3-prop-2-yloxyphenyl)-2-(3,5-dichloropyrid-4-yl)ethanone;
- EG: 1-(4-methoxy-3-prop-2-yloxyphenyl)-2-(3,5-dichloro-1-oxido-4-pyridinio)ethanone;
- EH: 1-(3-cyclopentyloxy-4-difluoromethoxyphenyl)-2-(3,5-dichloropyrid-4-yl)ethanone;
- EI: 1-(3-cyclopentyloxy-4-difluoromethoxyphenyl)-2-(3,5-dichloro-1-oxido-4-pyridinio)ethanone;
- EJ: 2-(3,5-dichloropyrid-4-yl)-1-[3-{exobicyclo(2.2.1)hept-5-en-2-yloxy}-4-methoxyphenyl]ethanone;
- EK: 2-(3,5-dichloro-4-pyridyl)-1-(4-difluoromethoxy-3-(exo)-8,9,10-trinorbom-2-yloxyphenyl)ethanone;
- EL: 2-(3,5-dichloro-1-oxido-4-pyridinio)-1-[4-difluoromethoxy-3-(exo)-8,9,10-trinorbom-2-yloxyphenyl]ethanone;
- EM: 2-(3,5-dichloro-4-pyridyl)-1-[4-methoxy-3-(3-methyl-2-butenyloxy)phenyl]ethanone;
- EN: 2-(3,5-dichloro-4-pyridyl)-1-(4-difluoromethoxy-3-isopropoxyphenyl)ethanone;
- EO: 2-(3,5-dichloro-1-oxido-4-pyridinio)-1-(4-difluoromethoxy-3-isopropoxyphenyl)ethanone;
and
- EP: 3,5-dichloro-4-(3-cyclopentyloxy-4-methoxyphenoxymethyl)pyridine;

Preferred compounds include CK, CL, EC, EJ, EK, EL and EQ.

The letters CJ to EQ are allocated to compounds for easy reference in this specification.

Compounds of formula I may be prepared by the application or adaptation of known methods, by which is meant methods used heretofore or described in the literature.

Thus, compounds of formula I, wherein R¹, R², R³, Z¹ and Z², are as hereinbefore defined, Z³ represents a -CZCH₂- linkage, and Z represents oxygen, are prepared from compounds of formula VI 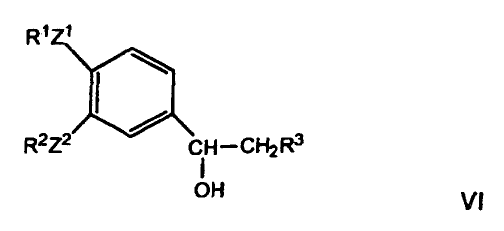 wherein R¹, R², R³, Z¹ and Z² are as hereinbefore defined, by oxidation by the application or adaptation of known methods. The oxidation is carried out, for example, by reaction with oxalyl chloride and dimethyl sulfoxide, in a solvent such as dichloromethane, and preferably at a temperature lower than -65°C. Alternatively, the oxidation is carried out by reaction with chromium trioxide in the presence of 3,5-dimethylpyrazole.

Compounds of formula I, wherein R¹, R², R³, Z, Z¹ and Z², are as hereinbefore defined, Z³ represents a -CZCH₂- linkage, and preferably those wherein Z represents oxygen, are prepared from compounds of formula VII 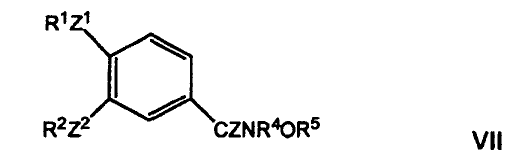 wherein R¹, R², Z, Z¹ and Z² are as hereinbefore defined and R⁴ and R⁵ represent lower alkyl, e.g. methyl, groups, by coupling with compounds of the formula VIII

R³CH₃ VIII

wherein R³ is as hereinbefore defined, in the presence of a strong base such as lithium diisopropylamide (usually prepared in situ from butyl lithium and diisopropylamine), preferably at a low temperature.

Compounds of formula I, wherein R¹, R², R³, Z¹ and Z², are as hereinbefore defined, Z³ represents a -CZCH₂- linkage, and Z represents oxygen, are prepared by the reaction of compounds of formula IX 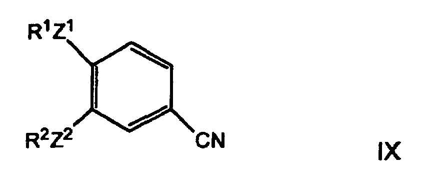 wherein R¹, R², Z¹ and Z² are as hereinbefore defined, with compounds of the formula X

R³MgX X

wherein R³ and X are as hereinbefore defined.

Alternatively, compounds of formula I, wherein R¹, R², R³, Z¹ and Z² are as hereinbefore defined and Z³ represents an -O-CH2- linkage are prepared by the reaction of compounds of the formula XI 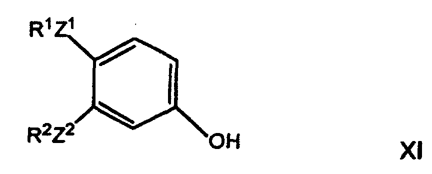 wherein R¹, R², Z¹ and Z² are as hereinbefore defined, with compounds of the formula XII

R³CH₂X² XII

wherein R³ and X are as hereinbefore defined, and X is preferably chloro, preferably takes place in the presence of a base such as an alkali metal carbonate, e.g. potassium carbonate, preferably in a solvent such as dimethylformamide.

Compounds of formula I, wherein R¹, R², R³, Z¹ and Z² are as hereinbefore defined and Z³ represents an -O-CO- linkage are prepared by the reaction of compounds of formula XI above, wherein R¹, R², Z¹ and Z² are as hereinbefore defined, with compounds of the formula XIII

R³COX XIII

wherein R³ and X are as hereinbefore defined, and X is preferably chloro, preferably in the presence of a base such as a tertiary amine, e.g. triethylamine, preferably in a solvent such as dichloromethane.

Compounds of formula I, wherein R¹, R², R³, Z¹ and Z² are as hereinbefore defined and Z³ represents an -NH-CO- linkage are prepared by the reaction of compounds of formula XIV 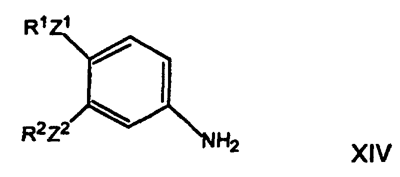 wherein R¹, R², Z¹ and Z² are as hereinbefore defined, with compounds of formula XIII above, wherein R³ and X² are as hereinbefore defined, preferably in the presence of a base such as a tertiary amine, e.g. triethylamine, preferably in a solvent such as dichloromethane.

Compounds of formula I, wherein R¹, R², R³, Z¹ and Z² are as hereinbefore defined and Z³ represents a -CH2-NH- linkage are prepared by the reaction of compounds of formula XVI 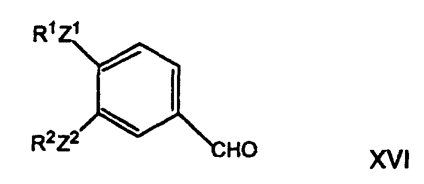 wherein R¹, R², Z¹ and Z² are as hereinbefore defined, with compounds of formula III

R³NH₂ III

wherein R³ is as hereinbefore defined, followed by reduction with a compound such as sodium cyanoborohydride. This process is especially suitable for compounds wherein R³ represents an optionally substituted phenyl or naphthyl group.

Compounds of formula I, wherein R¹, R², R³, Z¹ and Z² are as hereinbefore defined and Z³ represents a -CH2-NH- linkage are prepared by the reaction of compounds of formula XVII 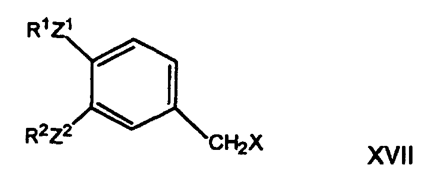 wherein X, R¹, R², Z¹ and Z² are as hereinbefore defined, and X is preferably bromo, with compounds of formula III above, wherein R³ is as hereinbefore defined, preferably in the presence of a base such as sodium hydride. This process is especially suitable for compounds wherein R³ represents an optionally substituted heteroaryl group.

Compounds of formula I, wherein R¹, R², R³, Z¹ and Z² are as hereinbefore defined and Z³ represents a trans -CH=CH- linkage are prepared by the reaction of compounds of formula XVI above, wherein R¹, R², Z¹ and Z² are as hereinbefore defined, with the reaction product of a compound of the formula XVIII

(R⁴PCH₂R³)⁺ (X)⁻ XVIII

(wherein R³ is as hereinbefore defined, R⁴ represents an aryl e.g. phenyl group, and X represents halo, preferably bromo) with a base such as an alkali metal alkoxide, e.g. potassium t-butoxide. The reaction is preferably carried out in a solvent such as tetrahydrofuran.

Compounds of formula I, wherein R¹, R², R³, Z¹ and Z² are as hereinbefore defined and Z³ represents an -S-CH2- linkage are prepared by the reaction of compounds of formula XX 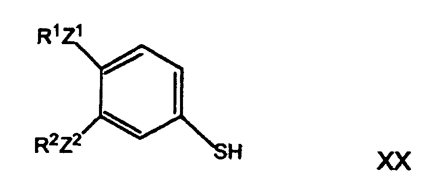 wherein R¹, R², Z¹ and Z² are as hereinbefore defined, with compounds of formula XII above, wherein R² and X are as hereinbefore defined, and preferably X is bromo, preferably after reaction with a base such as an alkali metal alkoxide, e.g. sodium methoxide.

Compounds of formula I, wherein R¹, R², R³, Z¹ and Z² are as hereinbefore defined and Z³ represents an -NH-CH₂- linkage are prepared by the reaction of compounds of formula XIV above, wherein R¹, R², Z¹ and Z² are as hereinbefore defined, with compounds of the formula XXIV

R³CHO XXIV

wherein R³ is as hereinbefore defined, preferably with the aid of a reducing agent such as sodium cyanoborohydride.

Compounds of formula I, wherein R¹, R², R³, Z¹ and Z² are as hereinbefore defined and Z³ represents a -CO-CO-NH- linkage are prepared by the reaction of compounds of formula XXIX 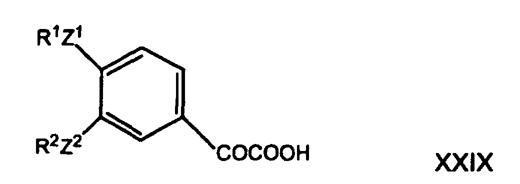 wherein R¹, R², Z¹ and Z² are as hereinbefore defined, with dichloromethyl methyl ether in dichloromethane, followed by reaction with compounds of formula III above, wherein R³ is as hereinbefore defined, preferably with the aid of a base such as sodium hydride, preferably in a solvent such as tetrahydrofuran.

Compounds of formula I, wherein R¹, R², R³, Z¹ and Z² are as hereinbefore defined and Z³ represents a -CO-CO- linkage are prepared by the oxidation of compounds of formula VI above, wherein R¹, R², R³, Z¹ and Z² are as hereinbefore defined, for example by reaction with pyridinium dichromate, preferably in a solvent such as dichloromethane. This reaction is particularly suitable for compounds wherein R³ represents a heteroaryl, preferably an optionally substituted pyridyl, group.

Compounds of formula I, wherein R¹, R², R³, Z¹ and Z² are as hereinbefore defined and Z³ represents a trans -N=N- linkage are prepared by the reaction of compounds of formula XXX 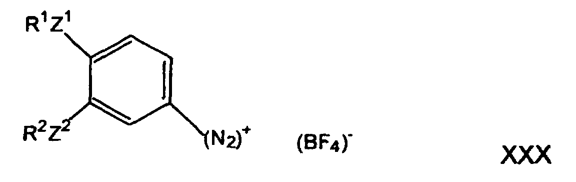 wherein R¹, R², R³, Z¹ and Z² are as hereinbefore defined, with compounds of the formula XXXI

R³H XXXI

wherein R³ is as hereinbefore defined, preferably with the aid of a base such as lithium diisopropylamide.

As another example, compounds of formula I wherein R¹, R², R3, Z¹ and Z² are as hereinbefore defined, and Z³ represents a cis -C=C- or cis -N=N-linkage are prepared by the action of ultraviolet radiation upon their trans-isomers.

As another example, compounds of formula I wherein R¹, R², R³, Z¹ and Z² are as hereinbefore defined, Z¹ and Z² preferably each represent oxygen, and Z³ represents an -SO-CH₂- linkage are prepared by the oxidation of corresponding compounds wherein Z³ represents an -S-CH₂- linkage. For example, the oxidation can be carried out by means of potassium hydrogen peroxomonosulfate in a medium such as aqueous methanol.

As another example, compounds of formula I wherein R¹, R², R3, Z¹ and Z² are as hereinbefore defined, Z¹ and Z² preferably each represent oxygen, and Z³ represents an -SO₂-CH₂- linkage are prepared by the oxidation of corresponding compounds wherein Z³ represents an -S-CH₂- linkage. For example, the oxidation can be carried out by means of sodium iodate in a medium such as aqueous methanol.

As another example, compounds of formula I wherein R¹, R², R3, Z¹ and Z² are as hereinbefore defined, Z³ represents a -CZCH₂- linkage, and Z represents sulfur are prepared from compounds of formula I wherein R¹, R², R3, Z¹ and Z² are as hereinbefore defined, Z³ represents a -CZCH₂- linkage, and Z represents oxygen, by reaction with phosphorus pentasulfide or 2,4-bis(4-methoxyphenyl)-1,3-dithia-2,4-diphosphetane-2,4-disulfide, preferably in a solvent such as pyridine or toluene, and preferably at a temperature from 0°C to the reflux temperature.

As another example, compounds of formula I wherein R¹, R², Z, Z¹, Z² and Z³ are as hereinbefore defined, Z, Z¹ and Z² preferably each represent oxygen, and R³ is as hereinbefore defined and contains an alkylsulfonyl, arylsulfonyl, alkylsulfinyl or arylsulfinyl group, are prepared by the oxidation of the corresponding compounds of formula I wherein R¹, R², Z, Z¹, Z² and Z³ are as hereinbefore defined and R³ is as hereinbefore defined and contains an alkylthio or arylthio group, preferably by means of reaction with a peroxyacid, e.g. 3-chloroperbenzoic acid, preferably in an inert solvent, e.g. dichloromethane, preferably at or near room temperature. Alternatively, the oxidation is carried out by reaction with a peroxomonosulfate, e.g. potassium peroxomonosulfate, conveniently in a solvent such as methanol, buffered to about pH 5, at temperatures between about 0°C and room temperature. This fatter method is preferred for compounds containing an acid-labile group, such as those wherein the moiety R²O- contains a carbon-carbon double bond between its beta- and gamma-carbon atoms, e.g. a cyclopent-2-enyloxy group.

As another example, compounds of formula I wherein R¹, R², Z, Z¹, Z² and Z³ are as hereinbefore defined, and Z is preferably oxygen, and R³ is as hereinbefore defined and contains a hydroxymethyl group are prepared by the reduction of the corresponding compounds of formula I wherein R¹, R2, Z¹, Z² and Z³ are as hereinbefore defined and R³ is as hereinbefore defined and contains an aryloxycarbonyl or, preferably, alkoxycarbonyl group, preferably by means of reaction with an alkali metal borohydride, preferably in an inert solvent, e.g. tetrahydrofuran, preferably at or near room temperature.

As another example, compounds of formula I wherein R¹, R², Z, Z¹, Z² and Z³ are as hereinbefore defined, Z preferably being an oxygen atom, and R3 is as hereinbefore defined and contains a formyl group are prepared by the oxidation of the corresponding compounds of formula I wherein R¹, R², Z, Z¹, Z² and Z³ are as hereinbefore defined and R³ is as hereinbefore defined and contains a hydroxymethyl group, for example by reaction with oxalyl chloride and dimethyl sulfoxide, in a solvent such as dichloromethane, and preferably at a temperature lower than -65°C, or, preferably, by reaction with a complex of sulfur trioxide with an amine such as pyridine, preferably in the presence of an amine such as triethylamine, preferably at or near room temperature.

As another example, compounds of formula I wherein R¹, R², Z, Z¹, Z² and Z³ are as hereinbefore defined, and Z is preferably oxygen, and R³ is as hereinbefore defined and contains an amino group are prepared by the reduction of the corresponding compounds of formula I wherein R¹, R², Z, Z¹, Z² and Z³ are as hereinbefore defined and R³ is as hereinbefore defined and contains a nitro group, preferably by means of reaction with iron in acidic conditions, e.g. in acetic acid, preferably at or above room temperature, more especially at the reflux temperature. Alternatively the reduction are carried out by reaction with hydrazine hydrate in the presence of ferric chloride and activated carbon, conveniently in a solvent such as methanol, at temperatures between about 25°C and 80°C. This latter method is preferred for compounds containing an acid-labile group, such as those wherein the moiety R²O-contains a carbon-carbon double bond between its beta and gamma-carbon atoms, e.g. a cyclopent-2-enyloxy group.

As another example, compounds of formula I wherein R¹, R², Z, Z¹, Z² and Z³ are as hereinbefore defined, and Z is preferably oxygen, and R³ is as hereinbefore defined and contains an alkanoylamino or aroylamino group are prepared from compounds of formula I wherein R¹, R², Z, Z¹, Z² and Z³ are as hereinbefore defined and R³ is as hereinbefore defined and contains an amino group, preferably by means of reaction with the appropriate acid halide or acid anhydride in the presence of a tertiary base such as triethylamine, optionally in an inert solvent, and preferably at a temperature from 0°C to the reflux temperature.

As another example, the compounds of formula I wherein R¹, R², Z, Z¹, Z² and Z³ are as hereinbefore defined, Z, Z¹ and Z² preferably are oxygen, and R³ represents a heteroaryl group containing one or more nitrogen ring atoms, can be converted to the corresponding N-oxides preferably by means of reaction with a mixture of hydrogen peroxide and an organic acid, e.g. acetic acid, preferably at or above room temperature at 60-90°C. Altematively, the oxidation is carried out by reaction with hydrogen peroxide in the presence of sodium tungstate at temperatures between room temperature and about 60°C. This latter method is preferred for compounds containing an acid-labile group, such as those wherein the moiety R²O- contains a carbon-carbon double bond between its beta- and gamma-carbon atoms, e.g. a cyclopent-2-enyloxy group.

For example, compounds of formula I wherein R¹ is as hereinbefore defined and is substituted on its alpha-carbon atom by fluorine and Z¹ is sulfur, and/or wherein R² is as hereinbefore defined and is substituted on its alpha-carbon atom by fluorine and Z² is sulfur, and R³ and Z³ as hereinbefore defined, are prepared by the reaction of xenon difluoride with corresponding compounds of formula I wherein said alpha-carbon atoms carry hydrogen atoms instead of said fluorine atoms. The reaction is conveniently carried out in a solvent, such as dichloromethane, in the presence of a molecular sieve, and in an inert atmosphere, at a low temperature, e.g. at or near 0°C.

As another example, compounds of formula I wherein R¹, R², Z, Z¹, Z² and Z³ are as hereinbefore defined, and R³ represents a heteroaryl group containing one or more nitrogen ring atoms but carrying no halogen substituents, are prepared by the reduction of the corresponding compounds of formula I wherein R³ does carry one or more halogen, e.g. chlorine, substituents, for example by means of ammonium formate in the presence of a palladium catalyst.

Compounds of the present invention may contain asymmetric centers. These asymmetric centers may independently be in either the R or S configuration. It will be apparent to those skilled in the art that certain compounds of the invention may also exhibit geometrical isomerism. The present invention comprises the individual geometrical isomers and stereoisomers and mixtures thereof.

Such isomers can be separated from their mixtures, by the application or adaptation of known methods, for example chromatographic techniques and recrystallization techniques, or they are separately prepared from the appropriate isomers of their intermediates, for example by the application or adaptation of methods described herein.

The compounds of the present invention are useful in the form of the free base or acid or in the form of a pharmaceutically acceptable salt thereof. All forms are within the scope of the invention.

Where the compound of the present invention is substituted with a basic moiety, acid addition salts are formed and are simply a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the patient in pharmaceutical doses of the salts, so that the beneficial inhibitory effects on TNF and PDE inherent in the free base are not vitiated by side effects ascribable to the anions. Although pharmaceutically acceptable salts of said basic compounds are preferred, all acid addition salts are useful as sources of the free base form even if the particular salt, per se, is desired only as an intermediate product as, for example, when the salt is formed only for purposes of purification, and identification, or when it is used as intermediate in preparing a pharmaceutically acceptable salt by ion exchange procedures. Pharmaceutically acceptable salts within the scope of the invention are those derived from the following acids: mineral acids such as hydrochloric acid, sulfuric acid, phosphoric acid and sulfamic acid; and organic acids such as acetic acid, citric acid, lactic acid, tartaric acid, malonic acid, methanesufonic acid, ethanesulfonic acid, benzenesulfonic acid, p-toluenesulfonic acid, cyclohexylsulfamic acid, quinic acid, and the like. The corresponding acid addition salts comprise the following: hydrohalides, e.g. hydrochloride and hydrobromide, sulfate, phosphate, nitrate, sulfamate, acetate, citrate, lactate, tartarate, malonate, oxalate, salicylate, propionate, succinate, fumarate, maleate, methylene-bis-B-hydroxynaphthoates, gentisates, mesylates, isethionates and di-p-toluoyltartratesmethanesulfonate, ethanesulfonate, benzenesulfonate, p-toluenesulfonate, cyclohexylsulfamate and quinate, respectively.

According to a further feature of the invention, acid addition salts of the compounds of this invention are prepared by reaction of the free base with the appropriate acid, by the application or adaptation of known methods. For example, the acid addition salts of the compounds of this invention are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

The acid addition salts of the compounds of this invention can be regenerated from the salts by the application or adaptation of known methods. For example, parent compounds of the invention can be regenerated from their acid addition salts by treatment with an alkali, e.g. aqueous sodium bicarbonate solution or aqueous ammonia solution.

Where the compound of the invention is substituted with an acidic moiety, base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the animal organism in pharmaceutical doses of the salts, so that the beneficial inhibitory effects on TNF and PDE inherent in the free acid are not vitiated by side effects ascribable to the cations. Pharmaceutically acceptable salts, including for example alkali and alkaline earth metal salts, within the scope of the invention are those derived from the following bases: sodium hydride, sodium hydroxide, potassium hydroxide, calcium hydroxide, aluminum hydroxide, lithium hydroxide, magnesium hydroxide, zinc hydroxide, ammonia, ethylenediamine, N-methyl-glucamine, lysine, arginine, omithine, choline, N,N'-dibenzylethylenediamine, chloroprocaine, disthanolamine, procaine, N-benzylphenethylamine, diethylamine, piperazine, tris(hydroxymethyl)-aminomethane, tetramethylammonium hydroxide, and the like.

Metal salts of compounds of the present invention may be obtained by contacting a hydride, hydroxide, carbonate or similar reactive compound of the chosen metal in an aqueous or organic solvent with the free acid form of the compound. The aqueous solvent employed may be water or it may be a mixture of water with an organic solvent, preferably an alcohol such as methanol or ethanol, a ketone such as acetone, an aliphatic ether such as tetrahydrofuran, or an ester such as ethyl acetate. Such reactions are normally conducted at ambient temperature but they may, if desired, be conducted with heating.

Amine salts of compounds of the present invention may be obtained by contacting an amine in an aqueous or organic solvent with the free acid form of the compound. Suitable aqueous solvents include water and mixtures of water with alcohols such as methanol or ethanol, ethers such as tetrahydrofuran, nitriles such as acetonitrile, or ketones such as acetone. Amino acid salts may be similarly prepared.

The base addition salts of the compounds of this invention can be regenerated from the salts by the application or adaptation of known methods. For example, parent compounds of the invention can be regenerated from their base addition salts by treatment with an acid, e.g. hydrochloric acid.

As will be self-evident to those skilled in the art, some of the compounds of this invention do not form stable salts. However, acid addition salts are most likely to be formed by compounds of this invention wherein R³ represents a nitrogen-containing heteroaryl group and/or wherein R³ contains an amino group as a substituent. Preferable acid addition salts of the compounds of the invention are those wherein R² is other than an acid labile group.

As well as being useful in themselves as active compounds, salts of compounds of the invention are useful for the purposes of purification of the compounds, for example by exploitation of the solubility differences between the salts and the parent compounds, side products and/or starting materials by techniques well known to those skilled in the art.

It will be apparent to those skilled in the art that certain compounds of formula I can exhibit isomerism, for example geometrical isomerism and optical isomerism. Geometrical isomers include the cis and trans forms of compounds of the invention having alkenyl or diazenyl moieties. All isomers within formula I, and their mixtures, are within the scope of the invention.

Such isomers can be separated from their mixtures, by the application or adaptation of known methods, for example chromatographic techniques and recrystallization techniques, or they are separately prepared from the appropriate isomers of their intermediates, for example by the application or adaptation of methods described herein.

The starting materials and intermediates are prepared by the application or adaptation of known methods, for example methods as described in the Reference Examples or their obvious chemical equivalents.

For example, compounds of formula II, wherein R¹, R², Z¹ and Z² are as hereinbefore defined, are prepared from compounds of formula XXXV 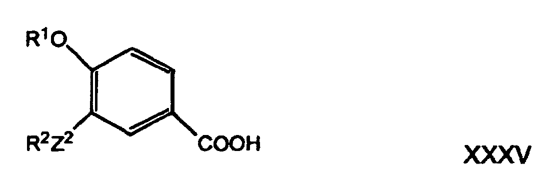 wherein R¹, R², Z¹ and Z² are as hereinbefore defined, by the application or adaptation of known methods for the preparation of acid halides from carboxylic, acids. For example, in compound of formula II 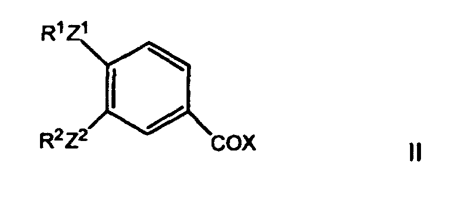 wherein R¹, R², Z¹ and Z² are as hereinbefore defined and X represents halo, e.g. bromo or, preferably, chloro, when X represents a chloro, the reaction can be carried out by means of thionyl chloride or, preferably, oxalyl chloride in the presence of triethylamine.

Compounds of formula XXXV, wherein R¹, R², Z¹ and Z² are as hereinbefore defined, are prepared by the oxidation of compounds of formula XVI above, wherein R¹, R², Z¹ and Z² are as hereinbefore defined, e.g. by means of reaction with potassium permanganate, or with a mixture of sulfamic acid and sodium chlorite in acetic acid, or with sodium chlorite in the presence of sodium dihydrogen phosphate.

Compounds of formula XVI, wherein R¹, R², Z¹ and Z² are as hereinbefore defined, are prepared from compounds of formula XXXVI 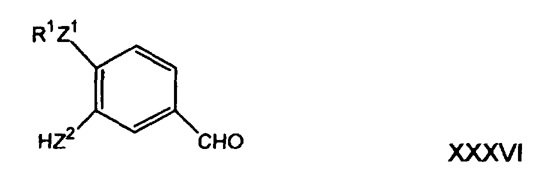 wherein R¹, Z¹ and Z² are as hereinbefore defined, by reaction with compounds of the formula:

R²X V

wherein R² and X are as hereinbefore defined, and X is preferably bromo, preferably in the presence of a base, for example an alkali metal hydride, e.g. sodium hydride, an alkali metal hydroxide or carbonate, e.g. sodium hydroxide or carbonate, or an amine, preferably a tertiary amine, e.g. triethylamine or pyridine, optionally in an inert solvent, for example dichloromethane, dimethylformamide, or an ether, e.g. diethyl ether or tetrahydrofuran, preferably at a temperature from 0°C to the reflux temperature, or alternatively by reaction with compounds of the formula XXXVII

R²OH XXXVII

wherein R² is as hereinbefore defined, preferably in the presence of a compound such as diisopropyl azodicarboxylate.

Alternatively compounds of formula XXXV above, wherein R¹, R², Z¹ and Z² are as hereinbefore defined, are prepared by the hydrolysis of compounds of formula XXXVIII 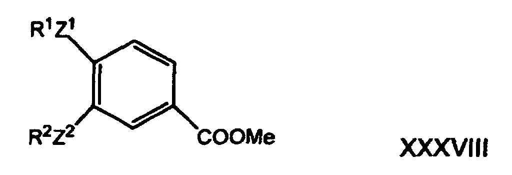 wherein R¹, R², Z¹ and Z² are as hereinbefore defined, e.g. by reaction with a base, such as an alkali metal carbonate or bicarbonate in the presence of water, followed by reaction with an aqueous acid such as dilute hydrochloric acid.

Compounds of formula XXXVIII above, wherein R¹, R², Z¹ and Z² are as hereinbefore defined, can be prepared from compounds of formula XXXIX 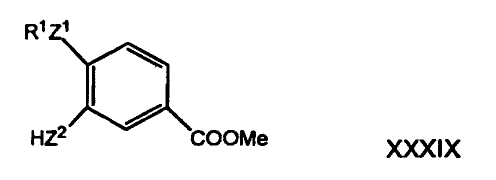 where R¹ is as herein before defined, by reaction with compounds of the formula XXXVII, wherein R² is as hereinbefore defined, preferably in the presence of diisopropyl azodicarboxylate and triphenylphosphine.

Compounds of formula VI above, wherein R¹, R², R³, Z¹ and Z² are as hereinbefore defined, are prepared by the reaction of compounds of formula XXXX 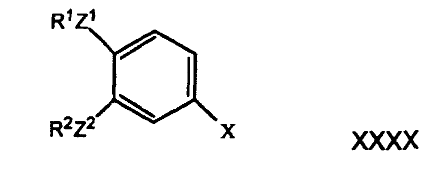 wherein R¹, R², Z¹ and Z² are as hereinbefore defined and X represents halo, e.g. bromo, with compounds of the formula XXXXI

R³CH2CHO XXXXI

wherein R³ is as hereinbefore defined, in the presence of a base such as butyl lithium, preferably at a low temperature.

Alternatively, compounds of formula VI above, wherein R¹, R², R³, Z¹ and Z² are as hereinbefore defined, are prepared by the reaction of compounds of formula XVI above, wherein R¹, R², Z¹ and Z² are as hereinbefore defined, with compounds of the formula VIII above,
wherein R³ is as hereinbefore defined, in the presence of a base such as lithium diisopropylamide (usually prepared in situ from butyl lithium and diisopropylamine), preferably at a low temperature.

Compounds of formula XXIX above, wherein R¹, R², Z¹ and Z² are as hereinbefore defined, are prepared by the reaction of selenium dioxide on the corresponding acetophenones in the presence of pyridine.

The present invention is further exemplified but not limited by the following illustrative examples which illustrate the preparation of the compounds according to the invention. The Reference Examples illustrate the preparation of the intermediates.

In the nuclear magnetic resonance spectra (NMR) the chemical shifts are expressed in ppm relative to tetramethylsilane. Abbreviations have the following significance: s=singlet; d=doublet; t=triplet; m=multiplet; dd=doublet of doublets; ddd=doublet of doublets of doublets; dt=doublet of triplets, b=broad.

### EXAMPLE 1 Compound CJ

A suspension of chromium trioxide (0.6 g) in dichloromethane (25 mL) is treated with 3,5-dimethylpyrazole (0.58 g), and stirred for 20 minutes. It is then treated with a solution of 1-(3-cyclopentyloxy-4-methoxyphenyl)-2-(2,6-dichlorophenyl)ethanol (1.4 g; that is prepared as described in Reference Example 12) in dichloromethane (10 mL) and the solution is stirred overnight at room temperature. It is then concentrated and the resulting residue is triturated with diethyl ether (200 mL) and filtered. The filtrate is evaporated to give a brown oil, which is subjected to flash chromatography, eluting with a mixture of diethyl ether and pentane (1:4 v/v), to give 3-cyclopentyloxy-4-methoxyphenyl 2',6'-dichlorobenzyl ketone (0.36 g), in the form of a white solid, m.p. 135-137°C. [NMR(CDCl₃): 7.73(dd,1H,J=8Hz,J=2Hz), 7.59(d,1 H,J=Hz), 7.35(d,2H,J=8Hz),7.18(t,1H,J=8Hz),6.94(d,1H,J=8Hz), 4.85(m,1H),4.66(s,2H),3.95(s,3H),2.05-1.52(m,8H). Elemental analysis: C,62.9,H,5.3%; calculated: C,63.3,H,5.3%].

### EXAMPLE 2 Compound CK

A solution of oxalyl chloride (5.3 mL) in dry dichloromethane (125 mL) at -60°C is treated portionwise with dimethyl sulfoxide (9.1 mL) in dichloro-methane (20 mL), keeping the temperature below -50°C. The solution is then stirred at -70°C for 20 minutes and is then treated with a suspension of 1-(3-cyclopentyloxy-4-methoxyphenyl)-2-(3,5-dichloropyrid-4-yl)ethanol (20.5 g; that is prepared as described in Reference Example 14) in dry dichloro-methane (300 mL) during 20 minutes, keeping the temperature below -50°C. After stirring for 30 minutes, the solution is treated with triethylamine (35 mL) and allowed to rise to room temperature. It is then treated with water (250 mL) and extracted with ethyl acetate (2 x 100 mL). The combined organic extracts are washed with dilute sulfuric acid (100 mL; 1%), aqueous potassium carbonate solution (100 ml; 5%) and brine (100 mL), dried and concentrated and the resulting residue is recrystallized from a mixture of ethyl acetate and heptane, to give 3-cyclopentyloxy-4-methoxyphenyl 3,5-dichloropyrid-4-ylmethyl ketone (19.6 g), m.p. 119-120°C. [NMR(CDCl₃): 8.52(s,2H),7.69 (dd, 1H,J=8Hz),7.57(d,1H,J=2Hz),6.95(d,1H,J=8Hz),4.86 (m,1H),4.64(s,2H), 3.95(s,3H),2.05-1.58(m,8H). Elemental analysis: C,59.8,H,4.95,N,3.63%; calculated: C,60.0,H,5.0,N,3.7%].

### Example 3 Compound CL

Aqueous 27.5% hydrogen peroxide (0.32 mL) is added to a solution of 3-cyclopentyloxy-4-methoxyphenyl 3,5-dichloropyrid-4-ylmethyl ketone (990 mg) in glacial acetic acid (13 mL). The reaction is heated at 80°C for 8 hours then allowed to stand overnight at room temperature. A further aliquot of aqueous 27.5% hydrogen peroxide (0.32 mL) is added and the mixture heated at 80°C for 2 hours. The reaction mixture is diluted with ethyl acetate (200 mL) and washed with saturated aqueous sodium bicarbonate until the washings remained basic. The mixture is washed with brine (50 mL), dried (MgSO₄), concentrated and the residue recrystallized from a mixture of dichloromethane/ethyl acetate/heptane to give 3,5-dichloro-4-(2-(3-cyclopentyloxy4-methoxyphenyl)-2-oxoethyl)pyridine-N-oxide as a yellow solid, m.p. 179-180°C. [Elemental analysis: C,57.3; H,4.81; N,3.54; Cl,18.0%; calculated for C₁₉H₁₉Cl₂NO₄: C,57.59; H,4.83; N,3.53; Cl,17.89%.]

### Example 4 Compound CM

A solution of diisopropylamine (1.23 mL) in dry tetrahydrofuran (15 mL) is stirred and cooled to -70°C under a nitrogen atmosphere. To this is added a 2.5 M solution of n-butyl lithium in hexanes (3.52 mL) at -70°C. The mixture is stirred for 30 minutes then a solution of 3-chloro-4-methylpyridine (1.02 g) in dry tetrahydrofuran (10 mL) is added. The mixture is stirred for a further 40 minutes. A solution of 3-cyclopentyloxy-4,N-dimethoxy-N-methylbenzamide (2.23 g) in dry tetrahydrofuran (10 mL) is added and the mixture stirred at -70°C for 30 minutes, -40°C for 30 minutes, 0°C for 30 minutes, and room temperature for 1 hour. A mixture of ethanol and hydrochloric acid 19:1 (40 mL) is added and then the reaction mixture is partitioned between brine (40 mL) and diethyl ether (40 mL). The ethereal phase is dried over sodium sulfate and concentrated in vacuo to give a pale yellow solid (3.0 g). The solid is triturated with diethyl ether and then purified by flash chromatography (ethyl acetate eluent on a silica column) to give a solid (1.6 g). The solid is triturated with diethyl ether, collected and dried to afford 1-(3-cyclopentyloxy-4-methoxyphenyl)-2-(3-chloropyrid-4-yl)ethanone (1.35 g) as a cream solid m.p. 124-125°C. [Elemental analysis: C,66.2; H,5.89; N,4.12%; calculated for C₁₉H₂₀ClNO₃: C,65.99; H,5.83; N,4.05%.]

### Example 5 Compound CN

5% Palladium on carbon (53 mg) is added to a solution of 3-cyclopentyloxy-4-methoxyphenyl 3,5-dichloropyrid-4-ylmethyl ketone (1.9 g) in hot methanol (60 mL) under a nitrogen atmosphere. The mixture is brought to reflux, ammonium formate (1.6 g) is added portionwise during 10 minutes and then refluxing is continued for a further 45 minutes. More 5% palladium on carbon (53 mg) and ammonium formate (1 g) are added and the mixture refluxed for 10 minutes. The reaction mixture is partitioned between dichloromethane (250 mL) and water (100 mL). The organic phase is separated, washed with water (75 mL) and brine (100 mL) and dried over magnesium sulfate. Evaporation yields a yellow gum (1.3 g) which is purified by flash chromatography (ethyl acetate/methanol 19: 1 v/v as eluent on a silica column) followed by recrystallization from cyclohexane to give 1-(3-cyclopentyloxy-4-methoxyphenyl)-2-(4-pyridyl)ethanone (0.55 g) as an off-white solid, m.p. 102-103°C. [Elemental analysis: C,72.6; H,6.63; N,4.23%; calculated for C₁₉H₂₁NO₃: C,73.29; H,6.80; N,4.50%.]

### Example 6 Compound CO (not part of the invention)

A solution of 3-cyclopentyloxy-4-methoxybenzonitrile (1.09 g) in dry tetrahydrofuran (3 mL) is added to a 2 M solution of benzylmagnesium chloride in tetrahydrofuran (5.0 mL) at room temperature under a nitrogen atmosphere. The mixture is refluxed for 3 hours, cooled in an ice bath and quenched with cold 4 M aqueous hydrochloric acid. More tetrahydrofuran (20 mL) is added and the reaction mixture is allowed to stand at room temperature for 48 hours. The tetrahydrofuran layer is decanted and evaporated. The residue is dissolved in cyclohexane (50 mL) and the solution washed successively with water (2x10 mL), 5% aqueous sodium bicarbonate (2 x 10 mL), water (2 x 10 mL) and brine (10 mL), and finally dried over magnesium sulfate. Concentration affords an amber oil (1.53 g) which is purified by flash chromatography (dichloromethane as eluant on silica column) to give a pale yellow viscous oil which crystallized on standing. The solid is recrystallized from methanol to afford 1-(3-cyclopentyloxy-4-methoxyphenyl)-2-phenylethanone as colorless crystals, m.p. 117-119°C. [Elemental analysis: C,77.7; H,7.2%; calculated for C₂₀H₂₂O₃: C,77.39; H,7.14%.]

### EXAMPLE 7 Compound CR

A stirred solution of 3-cyclopentyloxy-4-methoxyaniline (1 g) and triethylamine (0.69 mL) in dry dichloromethane (20 mL) at 0°C is treated dropwise with 2,6-dichlorobenzoyl chloride (1.17 g). After stirring at this temperature for 30 minutes, the mixture is warmed to room temperature and stirred for a further 3 hours. The organic layer is washed with water (100 mL), dried and concentrated. The residue is recrystallized from a mixture of isopropanol and hexane, to give N-(3-cyclopentyloxy-4-methoxyphenyl)-2,6-dichlorobenzamide (0.6 g), m.p. 184-185°C. [Elemental analysis: C,60.2,H,5.0; N,3.6; Cl,18.9%; calculated: C,60.0; H,5.0; N,3.7; Cl,18.65%].

### EXAMPLE 8 Compound CS

By proceeding in a similar manner to that described in Example 7, but using 2,6-difluorobenzoyl chloride, there is prepared N-(3-cyclopentyloxy-4-methoxyphenyl)-2,6-difluorobenzamide, m.p. 150-151 °C. [Elemental analysis: C,65.4; H,5.6; N,3.95;F,10.8%; calculated: C,65.7; H,5.5; N,4.0;F,10.9%].

### EXAMPLE 9 Compound CV

A stirred solution of 3-cyclopentyloxy-4-methoxyphenol (0.2 g) and triethylamine (1.35 mL) in dichloromethane (5 mL) is treated portionwise at 0-5°C with 2,6-dichlorobenzoyl chloride (0.28 g), and the solution is warmed to room temperature and stirred for a further 2 hours. The reaction mixture is poured into hydrochloric acid (50 ml; 2 N) and is extracted with diethyl ether (3 x 50 mL). The combined organic extracts are then washed with water (100 mL), and brine (100 mL), dried over magnesium sulfate and concentrated. The residual oil is subjected to flash chromatography on silica gel, eluting with a mixture of pentane and ethyl acetate (4:1 v/v), to give (3-cyclopentyloxy-4-methoxyphenyl) 2,6-dichlorobenzoate (0.28 g), m.p. 100-101°C. [Elemental analysis: C,59.7; H,4.7%; calculated: C,59.9; H,4.8%].

### EXAMPLE 10 Compound CW

A stirred solution of 3-cyclopentyloxy-4-methoxyphenol (0.5 g), potassium carbonate (0.4 g) and alpha 2,6-trichlorotoluene (0.56 g) in dimethylformamide (5 mL) is heated at 100°C for 1 hour. The solution is then concentrated and the residue is subjected to flash chromatography, eluting with a mixture of dichloromethane and pentane (1:1 v/v), to give 3-cyclopentyloxy-4-methoxyphenyl-2,6-dichlorobenzyl ether (0.76 g), m.p. 96-98°C. [Elemental analysis: C,61.7; H,5.5%; calculated: C,62.1; H,5.5%].

### EXAMPLE 11 Compound CX

A solution of 3-cyclopentyloxy-4-methoxybenzaldehyde (5 g) and 2-chloroaniline (2.5 mL) in toluene (60 mL) is heated at reflux under a Dean and Stark water trap for 3 hours. After concentration, the residue is dissolved in methanol (60 mL) and the stirred solution is treated at 0°C with sodium cyanoborohydride (2.1 g). The temperature is allowed to rise to room temperature, and the stirring is continued for 2 hours, before dilution with ethyl acetate (100 mL) and washing with saline (100 mL). The organic layer is dried and concentrated, to give a brown oil. This oil is subjected to flash chromatography on silica gel, eluting with a mixture of ethyl acetate and hexane (1:4v/v), to give N-(2-chlorophenyl)-3-cyclopentyloxy-4-methoxybenzylamine (0.64 g), in the form of an oil. [Elemental analysis: C,69.5; H,6.8; N,4.1; Cl,10.6%; calculated: C,68.8; H,6.7; N,3.2; Cl,10.7%].

### EXAMPLE 12 Compound CY

A stirred suspension of 2,6-dichlorobenzyltriphenylphosphonium bromide (2.5 g) in dry tetrahydrofuran (30 mL) is treated dropwise with a solution of potassium t-butoxide (0.56 g) in dry tetrahydrofuran (32 mL) at 0°C. After stirring at this temperature for 1 hour, it is treated with a solution of 3-cyclopentyloxy-4-methoxybenzaldehyde (1.1 g) in dry tetrahydrofuran (15 mL). The reaction mixture is stirred from 0°C to 5°C for 1 hour and 30 minutes, and then allowed to warm to room temperature. After stirring overnight, the mixture is concentrated and the resulting residue is treated with ethyl acetate (200 mL). The resulting organic solution is filtered. The filtrate is concentrated and the resulting residue is subjected to flash chromatography, eluting with dichloromethane, to give trans-1-(3-cyclopentyloxy-4-methoxyphenyl)-2-(2,6-dichlorophenyl)ethene (1.16 g), m.p. 47-49°C. [Elemental analysis: C,66.4; H,5.6; Cl,19.4%, calculated: C,66.1; H,5.55; Cl,19.5%].

### EXAMPLE 13 Compound CZ

By proceeding in a manner similar to that described in Example 14, but using 2,6-difluorobenzyltriphenylphosphonium bromide, there is prepared trans-1-(3-cyclopentyloxy-4-methoxyphenyl)-2-(2,6-difluorophenyl)ethene, m.p. 65-67°C. [Elemental analysis: C,73.0; H,6.1%; calculated: C,72.7; H,6.1%].

### EXAMPLE 14 Compound DA

Pyridinium dichromate (3.6 g) in dry dichloromethane (40 mL) under nitrogen is treated with (±)-1-(3-cyclopentyloxy-4-methoxyphenyl)-2-(pyrid-4-yl)ethanol (2.0 g; that is prepared as described in Reference Example 67), in one portion. The resulting mixture is stirred for 1 hour and 30 minutes, and then filtered through a pad of diatomaceous earth, and the pad is washed with diethyl ether. The combined filtrate and ethereal washings are washed with saturated aqueous cupric sulfate solution (2 x 30 mL), followed by water (30 mL), and then dried over magnesium sulfate. The solvent is removed under reduced pressure, and the resulting oily residue is subjected to flash chromatography on silica gel, eluting with ethyl acetate, to give 1-(3-cyclopentyloxy-4-methoxyphenyl)-2-(pyrid-4-yl)ethane-1,2-dione (0.4 g), in the form of a yellow solid, m.p. 117-119°C. [Elemental analysis: C,70.1; H,6.0; N,4.1 %; calculated: C,70.1; H,5.9; N,4.3%].

### EXAMPLE 15 Compound DB

A stirred solution of diisopropylamine (3.6 mL) in dry tetrahydrofuran (132 mL) is treated with a solution of butyl lithium in hexanes (10.3 mL; 2.5 M), dropwise, under nitrogen, keeping the temperature below -65°C. The resulting mixture is then stirred for a further period of 20 minutes, at below -65°C. The stirred mixture, still maintained at below -65°C, is then treated dropwise with a solution of 3,5-dichloropyridine (3.5 g) in dry tetrahydrofuran (24 mL). The stirred mixture is maintained at below -65°C for a further 30 minutes. The stirred mixture, still maintained at below -65°C, is then treated portionwise with 3-cyclopentyloxy-4-methoxyphenyldiazonium tetrafluoroborate (7.2 g), and it is stirred at below -65°C for a further 45 minutes. The resulting mixture is then allowed to warm to room temperature overnight, It is then treated with water (600 mL), the layers are separated, and the aqueous layer is further extracted with diethyl ether (3 x 100 mL). The combined organic extracts are washed with saturated aqueous sodium chloride solution (100 mL), dried over magnesium sulfate, and then evaporated to dryness. The resulting residue is subjected to flash chromatography on silica gel, eluting with a mixture of pentane and diethyl ether (2:1 v/v), to give a red solid (3.1 g) which, on recrystallization from pentane, gives trans-1-(3-cyclopentyloxy-4-methoxyphenyl)2-(3,5-dichloropyrid-4-yl)diazene (2.2 g), in the form of a red-brown solid, m.p. 88-89°C. [Elemental analysis: C,56.0; H,4.8; N,11.3%; calculated: C,55.75; H,4.7; N,11.5%].

### EXAMPLE 16 Compounds DC and DD

A solution of trans-1-(3-cyclopentyloxy-4-methoxyphenyl)-2-(3,5-dichloropyrid-4-yl)diazene (1.2 g; that is prepared as described in Example 15) in dichloromethane (24 mL) is treated portionwise with metachloroperbenzoic acid (0.6 g). The resulting mixture is stirred in the dark for 2 hours and 30 minutes, and then it is allowed to stand in the dark overnight. After the addition of a further quantity of dichloromethane (24 mL), the mixture is shaken with saturated aqueous sodium bicarbonate solution (12 mL). The layers are separated and the aqueous phase is further extracted with dichloromethane (3 x 6 mL). The combined organic extracts are washed with saturated aqueous sodium carbonate solution (6 mL), dried over magnesium sulfate, and evaporated to dryness. The resulting residual gum is dissolved in a mixture of dichloromethane and diisopropyl ether (1:2 v/v), and treated with activated carbon. After filtration, the solution is concentrated to low bulk. The resulting crystalline solid is filtered off and washed with diisopropyl ether and pentane, and dried in air. This material (0.71 g) is subjected to flash chromatography on silica gel, eluting initially with dichloromethane, and then with a mixture of dichloromethane and methanol (19:1 v/v), to give a solid (0.58 g), which is purified by reverse phase high pressure liquid chromatography on octadecylsilyl silica gel, eluting with a mixture of methanol and water (3:1 v/v).

1-(3-Cyclopentyloxy-4-methoxyphenyl)-c-1-oxo-r-2-(3,5-dichloro-1-oxopyrid-4-yl)diazene (0.17 g) is eluted first, in the form of a yellow solid, m.p. 139-141°C. [Elemental analysis: C,51.4; H,4.4; N,10.4%; calculated: C,51.3; H,4.3; N,10.6%].

Trans-1-(3-cyclopentyloxy-4-methoxyphenyl)-2-(3,5-dichloro-1-oxopyrid-4-yl)diazene (0.31 g) is eluted second, in the form of a red solid, m.p. 172-174°C [Elemental analysis: C,53.4; H,4.5; N,10.9%; calculated: C,53.4; H,4.5; N,11.0%].

### EXAMPLE 17 Compound EC

By proceeding in a manner similar to that described in Example 2, but using as the starting material the appropriate quantity of rac-1-[3-{(exo)-8,9,10-trinorbomyl-2-oxy}-4-methoxyphenyl]-2-(3,5-dichloropyrid-4-yl)ethanol there is prepared, after flash chromatography, eluting with a mixture of ethyl acetate and pentane (1:2 v/v), (±)-1-[3-{(exo)-8,9,10-trinorbomyl-2-oxy}-4-methoxyphenyl]-2-(3,5-dichloropyrid-4-yl)ethanone, in the form of a white solid, m.p. 113-114°C. [Elemental analysis: C,61.9; H,5.2; N,3.4%; calculated: C,62.1; H,5.2; N,3.4%].

### EXAMPLE 18 Compound ED

By proceeding in a manner similar to that described in Example 2, but using as the starting material the appropriate quantity of 1-[3-cyclopentyloxy-4-(methylthio)phenyl)]-2-(3,5-dichloropyrid-4-yl)ethanol, there is prepared 1-[3-cyclopentyloxy-4-(methylthio)phenyl]-2-(3,5-dichloropyrid-4-yl)ethanone in the form of a yellow solid, m.p. 110-111°C. [Elemental analysis: C,57.6; H,4.8; Cl,17.8; N,3.4;%; calculated: C,57.6; H,4.8; Cl,17.9; N,3.5%].

### EXAMPLE 19 Compound EE

By proceeding in a manner similar to that described in Example 2, but using as the starting material the appropriate quantity of 1-(4-methoxy-3-prop-2-yloxyphenyl)-2-(3,5-dichloropyrid-4-yl)ethanol, there is prepared 1-(4-methoxy-3-prop-2-yloxyphenyl)-2-(3,5-dichloropyrid-4-yl)ethanone, in the form of a buff solid, m.p. 153-155°C. [Elemental analysis: C,56.9; H,4.83; N,3.85%; calculated: C,57.64; H,4.84; N,3.95%].

### EXAMPLE 20 Compound EF

By proceeding in a manner similar to that described in Example 2, but using as the starting material the appropriate quantity of 1-(4-methylthio-3-prop-2-yloxyphenyl)-2-(3,5-dichloropyrid-4-yl)ethanol, there is prepared 1-(4-methylthio-3-prop-2-yloxyphenyl)-2-(3,5-dichloropyrid-4-yl)ethanone, in the form of a white solid, m.p. 116=117°C. [Elemental analysis: C,55.0; H,4.59; Cl,19.1; N,3.68; S,8.6%; calculated: C,55.14; H,4.63; Cl,19.2; N.3.78; S,8.7%].

### EXAMPLE 21 Compound EG

Hydrogen peroxide (8 mL) is added to a stirred suspension of 1-(4-methoxy-3-prop-2-yloxyphenyl)-2-(3,5-dichloropyrid-4-yl)ethanone (5.97 g) in glacial acetic acid (17 mL). The mixture is stirred at 70-80°C for 3 hours. After cooling, the mixture is basified by treatment with aqueous sodium hydroxide (6 M), and extracted with ethyl acetate. The extracts are washed with brine, dried over magnesium sulfate and evaporated, to give a white solid which is triturated with pentane and dried at 80°C, to give 1-(4-methoxy-3-prop-2-yloxyphenyl)-2-(3,5-dichloro-1-oxido-4-pyridinio)ethanone, in the form of a white solid, m.p. 167-169°C. [Elemental analysis: C,55.4; H,4.61; Cl,19.3; N,3.72%, calculated: C,55.15; H,4.63; Cl,19.2; N,3.78%].

### EXAMPLE 22 Compound EH

By proceeding in a manner similar to that described in Example 2, but using as the starting material the appropriate quantity of 1-(3-cyclopentyloxy-4-difluoromethoxyphenyl)-2-(3,5-dichloropyrid-4-yl)-ethanol, there is prepared 1-(3-cyclopentyloxy-4-difluoromethoxyphenyl)-2-(3,5-dichloropyrid-4-yl)ethanone, in the form of a white solid, m.p. 80-82°C. [Elemental analysis: C,55.1; H,4.1; N,3.2;%; calculated: C,54.8; H,4.1; N,3.4%].

### EXAMPLE 23 Compound EI

By proceeding in a manner similar to that described in Method A below, but using as the starting material the appropriate quantity of 1-(3-cyclopentyloxy-4-difluoromethoxyphenyl)-2-(3,5-dichloropyrid-4-yl)ethanone, there is prepared 1-(3-cyclopentyloxy-4-difluoromethoxy-phenyl)-2-(3,5-dichloro-1-oxido-4-pyridinio)ethanone, in the form of a white solid, m.p. 178-179°C. [Elemental analysis: C,53.1; H,4.1; N,3.1;%; calculated: C,52.8; H,4.0; N,3.2%].
Method A: A stirred suspension of N-(3,5-dichloropyrid-4yl)-3-cyclopentyloxy-4-methoxybenzamide (2.0 g; that is prepared as described in Method B below) in glacial acetic acid (8 mL) is treated with an aqueous solution of hydrogen peroxide (6 mL; 27.5%). The mixture is stirred for 3 hours at 70-80°C and then it is treated with a further portion of hydrogen peroxide solution (4 mL), and the solution is stirred for a further 12 hours. The solution is then cooled, basified by treatment with concentrated aqueous sodium hydroxide solution, and extracted with dichloromethane (2 x 30 mL). The organic extract is washed with brine (30 mL), dried over magnesium sulfate and evaporated. The resulting residue is recrystallized from ethyl acetate, to give 3,5-dichloro-4-(3-cyclopentyloxy-4-methoxybenzamido)pyridine-N-oxide (0.73 g), m.p. 118-120°C [Elemental analysis: C,53.0; H,4.4; N,6.8%; calculated for C₁₈H₁₈O₄N₂Cl₂:0.5H₂O: C,53.2;H 4.7;N 6.9%].
Method B: 4-Amino-3,5-dichloropyridine (4.0 g) and 3-cyclopentyloxy-4-methoxybenzoyl chloride (6.26 g) are intimately ground together in a mortar with a pestle, and transferred to a round-bottomed flask. The mixture is melted, using a hot air gun external to the flask, stirring with a magnetic stirrer. After 10 minutes, heating is ceased and the melt is allowed to cool. The resulting material is triturated with dichloromethane and the residual solid is filtered off. The filtrate is concentrated to give a fawn solid, which is subjected to flash chromatography on silica gel, eluting with diethyl ether, to give N-(3,5-dichloropyrid-4-yl)-3-cyclopentyloxy-4-methoxybenzamide (1.87 g), m.p. 155-157°C. [Elemental analysis: C,56.3; H,4.7; N,7.2; Cl,18.4%; calculated: C,56.7; H,4.76; N,7.35; Cl,18.6%; IR spectrum: 1661 cm⁻¹, 3244 cm⁻¹]

Alternatively, a suspension of 3-cyclopentyloxy-4-methoxybenzamide (2.58 g; that is prepared as described in Reference Example 19) in dry toluene (40 mL) is heated at reflux and treated with potassium t-butoxide (1.4 g), followed by 3,4,5-trichloro-pyridine (1.82 g). The mixture is then heated at reflux for 3 hours and 45 minutes, and is then treated with a further quantity of potassium t-butoxide (1.4 g) and heated at reflux for a further period of 7 hours. The mixture is allowed to cool and is then filtered. The filtrate is evaporated and the resulting residue is extracted with aqueous sodium hydroxide solution (2 M). The alkaline solution is then acidified by treatment with acetic acid, and the solid which separates is collected by filtration, washed with water and dried, to give N-(3,5-dichloropyrid-4-yl)-3-cyclopentyloxy-4-methoxy-benzamide (2.09 g) in the form of a buff solid, m.p. 153-155°C.

### EXAMPLE 24 Compound EJ

By proceeding in a manner similar to that described in Example 2, but using as the starting material the appropriate quantity of 2-(3,5-dichloro- pyrid-4-yl)-1-[3-{exobicyclo(2.2.1)hept-5-en-2-yloxy}-4-methoxyphenyl]ethanol, there is prepared 2-(3,5-dichloropyrid-4-yl)-1-[3-{exobicyclo(2.2.1)hept-5-en-2-yloxy}-4-methoxyphenyl]ethanone, in the form of a white solid, m.p. 89-91°C. [Elemental analysis: C,62.6; H,4.75; N,3.4%; calculated: C,62.4; H,4.7; N,3.5%].

### EXAMPLE 25 Compound EK

By proceeding in a manner similar to that described in Example 2, but using as the starting material the appropriate quantity of 2-(3,5-dichloro-4-pyridyl)-1-[4-difluoromethoxy-3-(exo)-8,9,10-trinorborn-2-yloxyphenyl]ethanol, there is prepared 2-(3,5-dichloro-4-pyridyl)-1-(4-difluoromethoxy-3-(exo)-8,9,10-trinorbom-2-yloxyphenyl)ethanone, in the form of a white solid, m.p. 120-122°C.

### EXAMPLE 26 Compound EL

By proceeding in a manner similar to that described in Example 23, Method A, but using as the starting material the appropriate quantity of 2-(3,5-dichloro-4-pyridyl)-1-[4-difluoromethoxy-3-(exo)-8,9,10-trinorborn-2-yloxyphenyl]ethanol, there is prepared 2-(3,5-dichloro-1-oxido-4-pyridinio)-1-[4-difluoromethoxy-3-(exo)-8,9,10-trinorbom-2-yloxyphenyl]ethanone, in the form of a white solid, m.p. 59-61°C.

### EXAMPLE 27 Compound EM

By proceeding in a manner similar to that described in Example 2, but using as the starting material the appropriate quantity of 2-(3,5-dichloro-4-pyridyl)-1-[4-methoxy-3-(3-methyl-2-butenyloxy)-phenyl]ethanol, there is prepared 2-(3,5-dichloro-4-pyridyl)-1-[4-methoxy-3-(3-methyl-2-butenyloxy)-phenyl]ethanone, in the form of a white solid, m.p. 115-117°C.

### EXAMPLE 28 Compound EN

By proceeding in a manner similar to that described in Example 2, but using as the starting material the appropriate quantity of 2-(3,5-dichloro-4-pyridyl)-1-(4-difluoromethoxy-3-isopropoxyphenyl)- ethanol, there is prepared 2-(3,5-dichloro-4-pyridyl)-1-(4-difluoromethoxy-3-isopropoxyphenyl)ethanone in the form of a white solid, m.p. 85-87°C. [Elemental analysis: C,52.7; H,3.85; N,3.6%; calculated: C,52.3; H,3.9; N,3.6%].

### EXAMPLE 29 Compound EO

By proceeding in a manner similar to that described in Example 23, Method A, but using as the starting material the appropriate quantity of 2-(3,5-dichloro-4-pyridyl)-1-(4-difluoromethoxy-3-isopropoxyphenyl)-ethanone there is prepared 2-(3,5-dichloro-1-oxido-4-pyridinio)-1-(4-difluoromethoxy-3-isopropoxyphenyl)-ethanone, in the form of a white solid, m.p. 138-140°C. [Elemental analysis: C,50.3; H,3.7; N,3.2; Cl,17.6%; calculated: C,50.2; H,3.7; N,3.45; Cl,17.45%].

### EXAMPLE 30 Compound EP

By proceeding in a manner similar to that described in Example 10, but using as the starting material the appropriate quantity of 4-bromomethyl-3,5-dichloropyridine, there is prepared 3,5-dichloro-4-(3-cyclopentyloxy-4-methoxyphenoxymethyl)pyridine, in the form of a white solid, m.p. 75-77°C.

### REFERENCE EXAMPLE 1

A stirred solution of 3-hydroxy-4-methoxybenzaldehyde (2.00 g) in dry dimethylformamide (20 mL) is treated portionwise with sodium hydride (60% dispersion in oil; 0.56 g) and the mixture is then heated for 1 hour at 50°C. It is then treated dropwise with cyclopentyl bromide (2.36 g) and is stirred and heated at 50°C for 22 hours. The solution is diluted with water (100 mL) and extracted with diethyl ether (2 x 100 mL). The ethereal extracts are combined, dried over magnesium sulfate and concentrated, to give 3-cyclopentyloxy-4-methoxybenzaldehyde (1.65 g) in the form of a golden oil.

### REFERENCE EXAMPLE 2

A stirred saturated aqueous solution of potassium permanganate (100 mL) is treated with 3-cyclopentyloxy-4-methoxybenzaldehyde (7.4 g; that is prepared as described hereinbefore in Reference Example 1) and sodium carbonate (3.4 g) and the mixture is stirred at 50°C for 1 hour, and then cooled to room temperature. The reaction mixture is acidified by treatment with concentrated hydrochloric acid and then it is treated with aqueous sodium bisulfite solution until a colorless solution is obtained. The reaction mixture is extracted with dichloromethane (2 x 100 mL) and the organic extracts are dried over magnesium sulfate and concentrated. The resulting residue is recrystallized from diethyl ether, to give 3-cyclopentyloxy-4-methoxybenzoic acid (4.78 g) in the form of white crystals. [NMR(CDCl₃): 1.7(s,2H),1.8-2.2 (m,6H),3.95(s,3H),4.85(s,1H),6.9(bs,1H)7.6(bs,1H), 7.8(s,1H),9.8(s,1H); Elemental analysis: C,65.6; H,6.8%; Calculated: C,66.1; H,6.8%].

### REFERENCE EXAMPLE 3

Stirring thionyl chloride (20 mL) is treated portionwise with 3-cyclopentyloxy-4-methoxybenzoic acid (5.0 g; that is prepared as described hereinbefore in Reference Example 2) and the solution is then heated at 85°C for 3 hours. Toluene (50 mL) is added and the mixture is concentrated to give 3-cyclopentyloxy-4-methoxybenzoyl chloride (4.12 g) in the form of an oil which slowly crystallized. [NMR(CDCl₃): 1.6-1.7 (m,2H),1.8-1.95(m,4H),1.94-2.05(m,2H),3.94(s,3H),4.85 (m,1H),6.9(d,1H),7.55(d,1H),7.8(q,1H); Elemental analysis: C,61.3; H,5.94; Cl,13.9%; Calculated: C,61.3; H,5.94; Cl,13.92%].

### REFERENCE EXAMPLE 4

A solution of triphenylphosphine (17.5 g) in dry tetrahydrofuran (50 mL) under nitrogen is treated with a solution of diisopropyl azodicarboxylate (13.5 g) in dry tetrahydrofuran (50 mL). The solution is stirred is treated with a solution of endo-8,9,10-trinorborneol (5.0 g) in dry tetrahydrofuran (50 mL) followed by a solution of 3-hydroxy-4-methoxybenzaldehyde (10.2 g) in dry tetrahydrofuran (50 mL). The solution is heated at reflux for 15 hours, cooled, poured into water (600 mL), and extracted with diethyl ether (300 mL). The extract is washed with water (100 mL), with aqueous sodium hydroxide solution (2 x 100 mL; 1 M) and with water (2 x 100 mL), dried over magnesium sulfate and evaporated, to give an oil, which is subjected to flash chromatography on silica gel, eluting with a mixture of pentane and ethyl acetate (95:5 v/v) to give 3-(exo-8,9,10-trinorbomyl-2-oxy)-4-methoxybenzaldehyde (8.2 g), m.p. 56-61°C.

### REFERENCE EXAMPLE 5

A solution of 3-hydroxy-4-methoxybenzaldehyde (14.20 g) in dry dimethylformamide (300 mL) is treated portionwise with sodium hydride (60% dispersion in oil; 3.70 g) at room temperature under nitrogen. 3-Chlorocyclopentene (9.6 mL) is added and the resulting mixture is stirred ovemight. The solvent is then removed under reduced pressure and the residue is partitioned between water (500 mL) and dichloromethane (500 mL) and the aqueous layer is further extracted with dichloromethane (500 mL). The combined organic extracts are dried and evaporated under reduced pressure and the residue is subjected to flash chromatography on silica gel, eluting with a mixture of ethyl acetate and pentane (1:1 v/v), to give 3-cyclopent-2-enyloxy-4-methoxybenzaldehyde, in the form of a pale brown oil (11.2 g).

### REFERENCE EXAMPLE 6

A stirred suspension of sodium hydride (60% in oil, 0.88 g) in dry dimethylformamide (44 mL) under nitrogen at between 5-10°C is treated with a solution of 3-hydroxy-4-methoxy benzaldehyde (3.35 g) in dry dimethylformamide (6.3 mL). The resulting mixture is allowed to warm to room temperature and stirred for 40 minutes before recooling to between 5-10°C. A solution of 4-(p-toluenesulfonoxy)cyclopentene (5.24 g) in dry dimethylformamide (12.6 mL) is added dropwise maintaining the temperature below 10°C. The resulting mixture is allowed to warm to room temperature, left to stand for 46 hours, and then poured into 5% aqueous potassium carbonate (305 mL). t-Butyl methyl ether is added (150 mL), and the layers are thoroughly stirred and separated. The aqueous layer is further extracted with t-butyl methyl ether (2 x 75 mL), the combined organic extracts are washed with water (3 x 30 mL) and dried over magnesium sulfate. The solvent is removed under reduced pressure and the resulting residue is subjected to flash chromatography on silica gel, eluting with mixtures of ethyl acetate and pentane (1:10 to 3:10), to give 3-cyclopent-3-enyloxy-4-methoxybenzaldehyde as a pale amber viscous oil that slowly crystallizes on standing (1.75 g). Recrystallization of a portion (0.5 g) from cyclohexane gives an analytically pure sample (0.4 g), m.p. 60-62°C [Elemental analysis: C,71.8; H,6.5%; calculated: C,71.5; H,6.8%].

### REFERENCE EXAMPLE 7

A stirred solution of sodium hydroxide (16.8 g) in water (32 mL) at 20°C is treated with dimethyl sulfoxide (560 mL). It is then treated with 3,4-dihydroxybenzaldehyde (56.9 g), portionwise during 5 minutes, while keeping the temperature at 20°C. It is then treated with benzyl bromide (49.7 mL), portionwise, at 20°C. The solution is then heated at 80°C for 6 hours and then allowed to stand at room temperature overnight. After dilution with ice-water (2240 mL) the solution is extracted with diethyl ether (1 x 1000 mL, 2 x 250 mL). The combined ether extracts are washed with water, dried over magnesium sulfate and concentrated, to give an oily solid, which is recrystallized from a mixture of ethyl acetate and isopropanol, to give 4-benzyloxy-3-hydroxybenzaldehyde (60.9 g), in the form of pale yellow crystals, m.p. 118-120°C.

### REFERENCE EXAMPLE 8

A stirred solution of 4-benzyloxy-3-hydroxybenzaldehyde (60.9 g; that is prepared as described in Reference Example 7) in dry dimethylformamide (270 mL) under nitrogen is treated portionwise with potassium carbonate (79.5 g). After stirring at room temperature for 45 minutes, it is treated with cyclopentyl bromide (34.3 mL), and the resulting suspension is heated at 60°C for 8 hours. After cooling, the solution is evaporated to low bulk under reduced pressure, to give an oil. This oil is treated with water (250 mL) and diethyl ether (300 mL), and the aqueous layer is washed with further quantities of diethyl ether (2 x 50 mL). The combined ethereal extracts are washed with brine (1 x 50 mL) and with water (3 x 50 mL), dried over magnesium sulfate and evaporated. The resulting residue is crystallized from methanol, to give 4-benzyloxy-3-cyclopentyloxybenzaldehyde (79 g), m.p. 55-56°C. [Elemental analysis: C,77.1; H,6.9%; calculated: C,77.0; H,6.8%].

### REFERENCE EXAMPLE 9

A suspension of 2-methoxyphenylbenzoate (212.5 g) in glacial acetic acid (1 L) is treated dropwise during 1 hour with a solution of bromine (51.5 mL) in glacial acetic acid (150 mL). The mixture is stirred for a further 1 hour, then it is concentrated and the residue is dissolved in t-butyl methyl ether (1500 mL). The solution is washed with water (500 mL) and with saturated aqueous sodium bicarbonate solution. The solution is then dried, filtered and concentrated, to give 2-benzoyloxy-4-bromoanisole (205.8 g), in the form of a white solid, m.p. 73-75°C.

### REFERENCE EXAMPLE 10

A solution of 2-benzoyloxy-4-bromoanisole (5 g) and sodium hydroxide (3 g) in water (5 mL) and ethanol (50 mL) is heated at reflux for 1 hour 30 minutes. It is then evaporated and the residue is triturated with water (20 mL) and concentrated hydrochloric acid (10 mL) and extracted with dichloromethane (150 mL). The organic solution is washed with saturated aqueous sodium bicarbonate solution (3 x 25 mL), dried, and concentrated, to give 5-bromo-2-methoxyphenol (3.25 g), in the form of a white crystalline solid, m.p. 67-68°C.

### REFERENCE EXAMPLE 11

A stirred solution of 5-bromo-2-methoxyphenol (74 g) and anhydrous potassium carbonate (73.6 g) in dry dimethylformamide (500 mL) is treated with cyclopentyl bromide (80.5 g) and the solution is heated at 60°C for 16 hours. It is then concentrated, and the resulting residue is triturated with water (250 mL), and extracted with dichloromethane (3 x 250 mL). The combined extracts are dried and evaporated, to give 3-cyclopentyloxy-4-methoxyphenyl bromide (95.5 g), in the form of a light brown oil.

### REFERENCE EXAMPLE 12

A solution of butyl lithium in hexane (5.1 mL; 2.5 M) is treated with a solution of 3-cyclopentyloxy-4-methoxyphenyl bromide (3.45 g) in dry tetrahydrofuran (30 mL) at -70°C and the solution is then stirred at -70°C for 1 hour. It is then treated dropwise with 2,6-dichlorophenylacetaldehyde (2.4 g), while keeping the temperature below -60°C. When the addition is complete the temperature is allowed to rise to room temperature and the solution is stirred for a further 2 hours. The reaction mixture is treated with aqueous ammonium chloride solution (50 mL) and the solution is extracted with diethyl ether (2 x 200 mL). The combined extracts are dried and concentrated, to give a yellow oil, which is subjected to flash chromatography, eluting with a mixture of diethyl ether and pentane (1:8 v/v), to give 1-(3-cyclopentyloxy-4-methoxyphenyl)-2-(2,6-dichlorophenyl)ethanol (1.6 g),in the form of a white solid, m.p. 87-89°C.

### REFERENCE EXAMPLE 13

A solution of diisopropylamine (10.5 mL) in dry tetrahydrofuran (150 mL) is cooled to -75°C and treated with a solution of butyl lithium in hexane (30 mL; 2.5 M) during 10 minutes. After stirring for 1 hour at -75°C the mixture is treated with a solution of 3,5-dichloropyridine (10.8 g) in dry tetrahydrofuran (55 mL) and stirred for a further 30 minutes. It is then treated with methyl iodide (4.7 mL) in dry tetrahydrofuran (10 mL) during 10 minutes, and the solution is allowed to rise gradually to room temperature. After stirring for 2 hours, the mixture is treated with saturated aqueous sodium bicarbonate solution (50 mL), followed by diethyl ether (100 mL). The organic layer is washed with brine, dried and evaporated, and the resulting residue is subjected to flash chromatography, to give 3,5-dichloro-4-methylpyridine (10.6 g), m.p. 46-47°C.

### REFERENCE EXAMPLE 14

A stirred solution of diisopropylamine (9.75 mL) in dry tetrahydrofuran (200 mL) at -70°C is treated dropwise with a solution of butyl lithium in hexane (27.2 mL; 2.5 M) and the solution is stirred for 30 minutes. It is then treated with a solution of 3,5-dichloro-4-methylpyridine (10.25 g) in dry tetrahydrofuran (60 mL) during 30 minutes, while keeping the temperature below -75°C, and the solution is then stirred for a further 30 minutes. It is then treated with 3-cyclopentyloxy-4-methoxybenzaldehyde (13.92 g) in dry tetrahydrofuran (60 mL) during 15 minutes, and stirred for a further 1 hour 30 minutes at -75°C. The solution is treated with aqueous ammonium chloride solution and extracted with ethyl acetate (3 x 100 mL). The organic layers are combined, washed with brine, dried and concentrated to low volume. The resulting precipitate is filtered off, to give 1-(3-cyclopentyloxy-4-methoxyphenyl)-2-(3,5-dichloropyrid-4-yl)ethanol (20.5 g), m.p. 124-125°C.

### REFERENCE EXAMPLE 15

Oxalyl chloride (2 mL) is added to a stirred solution of 3-cyclopentyloxy-4-methoxybenzoic acid (2.36 g) in dry dichloromethane (50 mL) under a nitrogen atmosphere, and the mixture stirred for 3 hours. The reaction mixture is concentrated in vacuo and the residue dissolved in fresh dry dichloromethane (50 mL). N,O-dimethylhydroxylamine hydrochloride (1.12 g) and 2,4,6-collidine (2.9 mL) are added and the mixture stirred for 4 hours. The mixture is diluted with dichloromethane (100 mL) and washed with saturated aqueous sodium bicarbonate (2 x 30 mL), 2 M aqueous hydrochloric acid (2 x 30 mL) and water (20 mL). The dried (MgSO₄) solution is concentrated to an orange oil (3 g) which is purified by flash chromatography (gradient elution ethyl acetate/pentane (2:3 v/v) to ethyl acetate/pentane (2:1 v/v) on silica column) to give 3-cyclopentyloxy-4,N-dimethoxy-N-methylbenzamide (2.5 g) as a yellow syrup.

### REFERENCE EXAMPLE 16

Hydroxylamine hydrochloride (3.82 g) is added to a solution of 3-cyclopentyloxy-4-methoxybenzaldehyde (11.0 g) in acetic anhydride (30 mL) and the suspension heated in an oil bath at 100°C until refluxing starts. The heating is then removed while the exothermic reaction refluxes gently. Heating at 100°C is then continued for a further 1 hour. The dark solution is concentrated and the crude product dissolved in cyclohexane (200 mL). The solution is washed with 5% sodium bicarbonate solution (2 x 50 mL), water (2 x 50 mL) and brine (50 mL), and dried over magnesium sulfate. Upon evaporation, an amber oil (11.8 g) that is purified by flash chromatography (dichloromethane as eluant on silica column) gives 3-cyctopentyloxy-4-methoxybenzonitrile (8.6 g) as a pale yellow oil. [Elemental analysis: C,71.7; H,6.93; N,6.47%; calculated for C₁₃H₁₅NO₂: C,71.87; H,6.96; N,6.45%.].

### REFERENCE EXAMPLE 17

A stirred solution of 5-amino-2-methoxyphenol (10 g) in dry dioxane (150 mL) is treated portionwise with an oil suspension of sodium hydride (60%; 3 g; 75 mmol) and the mixture is then warmed at 60°C for 30 minutes. It is then treated dropwise with a solution of cyclopentyl bromide (9.2 mL) and potassium iodide (50 mg) in dry dimethylformamide (20 mL) and heated at reflux for 5 hours. The mixture is then concentrated and the residue is treated with ethyl acetate (200 mL) and water (200 mL). The organic layer is then separated, washed with water (100 mL), aqueous sodium hydroxide solution (250 mL;1 N), and with water (100 mL), and dried over magnesium sulfate. The concentration of the reaction mixture gives a dark oil, which is subjected to flash chromatography, eluting with a mixture of n-hexane and ethyl acetate (1:1 v/v), to give 3-cyclopentyloxy-4-methoxyaniline (4.43 g), in the form of an oil.

### REFERENCE EXAMPLE 18

A stirred solution of diisopropylamine (14 mL) in dry tetrahydrofuran (150 mL), is treated dropwise with a solution of butyl lithium in hexanes (40 mL; 2.5 M), under nitrogen, while keeping the temperature at below -70°C. The resulting mixture is then stirred for a further period of 30 minutes at below -70°C. The stirred mixture, while it is still maintained at below -70°C, is then treated dropwise with a solution of 4-picoline (9.3 g) in dry tetrahydrofuran (20 mL). The stirred mixture is maintained at below -70°C for a further 45 minutes. The stirred mixture, while it is still maintained at below -70°C, is then treated with a solution of 3-cyclopentyloxy-4-methoxybenzaldehyde (22.0 g) in dry tetrahydrofuran (100 mL), and it is stirred at below -70°C for a further 30 minutes. The resulting mixture is then allowed to warm to room temperature ovemight, and then treated with saturated aqueous ammonium chloride solution (200 mL). The layers are separated and the aqueous layer is further extracted with ethyl acetate (3 x 300 mL). The combined organic extracts are dried over magnesium sulfate and evaporated to dryness. The resulting residue is recrystallized from ethyl acetate, to give (±)-1-(3-cyclopentyloxy-4-methoxyphenyl)-2-(pyrid-4-yl)ethanol (28.5 g), in the form of a cream solid, m.p. 102-103°C.

### REFERENCE EXAMPLE 19

A stirred solution of concentrated hydrochloric acid (1.7 mL) in water (5 mL) from 0°C and 5°C is treated with 3-cyclopentyloxy-4-methoxyaniline (0.83 g), followed by a solution of sodium nitrite (0.29 g) in water (0.6 mL), at such a rate that the temperature remains from 0°C and 5°C. The resulting solution is stirred for a further 10 minutes, while keeping the temperature below 5°C. The stirred solution, while still maintained at below 5°C, is then treated dropwise with a solution of sodium tetrafluoroborate (0.88 g) in water (1.8 mL). The resulting precipitate is filtered off, washed with cold water (3 mL) and dried in vacuo, to give 3-cyclopentyloxy-4-methoxyphenyldiazonium tetrafluoroborate (1.24 g) in the form of a gray solid, m.p. 120°C.

### REFERENCE EXAMPLE 20

Vigorously stirred aqueous ammonia solution (70 mL; 32% w/w) is treatec dropwise with warm molten 3-cyclopentyloxy-4-methoxybenzoyl chloride (25.7 g) during a period of 20 minutes, keeping the temperature below 20°C. Stirring is continued at 20°C for 2 hours, and then the suspension is filtered. The resulting solid is washed with water until free of ammonia, and is then dried under vacuum at 40-45°C, to give 3-cyclopentyloxy-4-methoxy- benzamide (21.78 g), in the form of a buff solid.

### REFERENCE EXAMPLE 21

A stirred solution of 3-hydroxy-4-methoxy- benzaldehyde (5.74 g) in dry dimethylformamide (50 mL) is treated with cyclopentylmethyl bromide (7.34 g) and potassium carbonate (15 g), and the solution is heated at 60°C for 24 hours. After cooling and filtration, the solution is evaporated to low bulk and dissolved in ethyl acetate (100 mL). The organic solution is washed with aqueous sodium hydroxide solution (4 x 50 mL; 2 N) and water (2 x 50 mL), dried over magnesium sulfate, and evaporated to give 3-cyclopentyl- methoxy-4-methoxybenzaldehyde (6.5 g) in the form of a light brown oil.

By proceeding in a similar manner, but using the appropriate quantity of isopropyl bromide, there is prepared 3-isopropoxy-4-methoxybenzaldehyde, in the form of a light golden oil. [NMR (CDCl3): 9.85(s,1H), 7.46(dd,1H), 7.43(d,1H), 6.98(d,1H), 4.65(m1H), 3.94(s,3H), 1.41(d,6H)].

### REFERENCE EXAMPLE 22

By proceeding in a manner similar to that described in Reference Example 1, but using the appropriate quantity of cyclopropylmethyl bromide, there is prepared 3-cyclopropylmethoxy-4-methoxy-benzaldehyde, m.p. 55-58°C. [Elemental analysis: C,70.0; H,6.85%; calculated: C,69.9; H,6.8%].

By proceeding in a similar manner, but using the appropriate quantity of 3-bromopentane, there is prepared 4-methoxy-3-(pent-3-yloxy)benzaldehyde, in the form of a golden oil.

### REFERENCE EXAMPLE 23

By proceeding in a similar manner to Reference Example 4, but using 2-trans-fluorocyclopentanol as the starting material, there is prepared 3-(2-fluorocyclopentyloxy)-4-methoxybenzaldehyde. Mass spectrum m/z 238(M⁺).

### REFERENCE EXAMPLE 24

By proceeding in a manner similar to that described in Reference Example 14, but using as the starting material the appropriate quantity of (±)-3-[(exo)-8,9,10-trinorbornyl-2-oxy]-4-methoxybenzaldehyde, there is prepared, after flash chromatography, eluting with a mixture of ethyl acetate and pentane (1:2v/v), rac-1-[3-{(exo)-8,9,10-trinorbornyl-2-oxy}-4-methoxyphenyl]-2-(3,5-dichloropyrid-4-yl)ethanone, in the form of a yellow oil.

### REFERENCE EXAMPLE 25

By proceeding in a manner similar to that described in Reference Example 14, but using as the starting material the appropriate quantity of 3-cyclopentyloxy-4-(methylthio)benzaldehyde, there is prepared 1-[3-cyclopentyloxy-4-(methylthiophenyl)-2-(3,5-dichloropyrid-4-yl)ethanol, in the form of a white solid, m.p. 122-123°C. [Elemental analysis: C,57.3; H,5.3; Cl,17.6; N,3.4; S,8.4%; calculated: C,57.3; H,5.3; Cl,17.8; N,3.5; S,8.1%].

### REFERENCE EXAMPLE 26

A solution of 4-bromo-2-cyclopentyloxy-1-(methylthio)benzene (13.2 g) in dry tetrahydrofuran (100 mL) under nitrogen is treated at -70°C with a solution of butyllithium in hexane (20.24 mL; 2.5 M) and the resulting solution is stirred for 1 hour at this temperature. It is then treated with dimethylformamide (7.12 mL), followed by boron trifluoride diethyl etherate (11.32 mL), keeping the temperature at -65°C. When the addition is complete the reaction mixture is allowed to warm to room temperature and is then poured into ice- water (450 mL) and extracted with dichloromethane (3 x 400 mL). The combined extracts are dried over magnesium sulfate and concentrated to give an oil, which is subjected to flash chromatography, eluting with a mixture of dichloromethane and cyclohexane (3:7 v/v), to give 3-cyclopentyloxy-4-(methylthio)-benzaldehyde (6.2 g). [NMR (DMSO): 9.91(S,1H),7.53(dd,1H),7.36(d,1H), 7.33(d,1 H),5.01 (m,1H),2.44(s,3H),2.0-1.5(m,8H)].

### REFERENCE EXAMPLE 27

A stirred solution of 4-bromo-2-hydroxythioanisole (15.9 g) and cyclopentyl bromide (16.05 g) in dry dimethylformamide (170 mL) is treated with anhydrous potassium carbonate (14.7 g) and the mixture is heated at 60°C for 1 day. After cooling, the solution is diluted with water (250 mL) and extracted with ethyl acetate (3 x 100 mL). The combined extracts are dried over magnesium sulfate and concentrated. The residue is subjected to flash chromatography, eluting with a mixture of ethyl acetate and pentane (5:95 v/v), to give 4-bromo-2-cyclopentyloxy-1-(methylthio)benzene, in the form of an oil (17.2 g). [NMR (DMSO): 7.14-7.03(m,3H),4.93(m,1H), 2.33(s,3H),2.0-1.53(m,8H)].

### REFERENCE EXAMPLE 28

By proceeding in a manner similar to that described in Reference Example 14, but using as the starting material the appropriate quantity of 4-methoxy-3-prop-2-yloxybenzaldehyde, there is prepared 1-(4-methoxy-3-prop-2-yloxyphenyl)-2-(3,5-dichloropyrid-4-yl)ethanol, in the form of a buff solid, m.p. 132-133°C. [Elemental analysis: C,57.2; H,5.37; Cl,19.8; N,3.80%; calculated: C,57.32; H,5.38; Cl,19.9; N,3.93%].

### REFERENCE EXAMPLE 29

A mixture of 3-hydroxy-4-methoxybenzaldehyde (20.0 g), anhydrous potassium carbonate (26.2 g) and 2-bromopropane (18.3 mL) in dry dimethylformamide (300 mL) is stirred and heated at 55-65°C for 24 hours. The cooled mixture is poured into water and extracted with ethyl acetate. The extract is dried over magnesium sulfate and concentrated in vacuo at 40°C, to give 4-methoxy-3-prop-2-yloxybenzaldehyde, in the form of an oil (24.2 g).

### REFERENCE EXAMPLE 30

By proceeding in a manner similar to that described in Example 14, but using as the starting material the appropriate quantity of 4-methylthio-3-prop-2-yloxybenzaldehyde, there is prepared 1-(4-methylthio-3-prop-2-yloxyphonyl)-2-(3,5-dichloropyrid-4-yl)ethanol, in the form of a buff solid, m.p. 106-108°C. [Elemental analysis: 0,55.1; H,5.23; N,3.73%; calculated: C,54.84; H,5.14; N,3.76%].

### REFERENCE EXAMPLE 31

A solution of n-butyllithium in hexanes (14.82 mL; 2.5M) is added to a solution of 4-bromo-2-prop-2-yloxythioanisole (8.8 g) in dry tetrahydrofuran (70 mL), whilst cooling the mixture to -70°C under nitrogen. The mixture is stirred for 1 hour at -70°C. Dry dimethylformamide (5.22 mL) and boron trifluoride diethyl etherate (8.29 mL) are added sequentially and the mixture is allowed to warm to room temperature. The mixture is poured into water and the product is extracted with dichloromethane. The extract is dried over magnesium sulfate and concentrated in vacuo, and the residue is subjected to flash chromatography on silica gel, eluting with a mixture of pentane and ethyl acetate (9:1 v/v) to give 4-methylthio-3-prop-2-yloxybenzaldehyde, in the form of a soft yellow solid, m.p. 50-54°C.

### REFERENCE EXAMPLE 32

By proceeding in a manner similar to that described in Reference Example 26, but using as the starting material the appropriate quantity of 2-bromopropane, there is prepared 4-bromo-2-prop-2-yloxythioanisole, in the form of a colourless oil.

### REFERENCE EXAMPLE 33

By proceeding in a manner similar to that described in Reference Example 14, but using as the starting material the appropriate quantity of 3-cyclopentyloxy-4-difluoromethoxybenzaldehyde, there is prepared 1-(3-cyclopentyloxy-4-difluoromethoxyphenyl)-2-(3,5-dichloropyrid-4-yl)ethanol, in the form of a pale yellow solid, m.p. 121-123°C. [Elemental analysis: C,55.2; H,4.6; N,3.3; Cl,16.7%; calculated: C,54.6; H,4.6; N,3.35; Cl,16.95%].

### REFERENCE EXAMPLE 34

A solution of 3-cyclopentyloxy-4-hydroxybenzaldehyde (5.1 g) in dimethylformamide (50 mL) is treated with potassium carbonate (4.83 g) and potassium iodide (1.2 g) and the solution is heated at 70-75°C whilst difluorochloromethane is bubbled though it at a slow rate, during 5 hours. Water (100 mL) is added and the mixture is extracted with ethyl acetate (2 x 100 mL). The combined organic extracts are dried over magnesium sulfate and concentrated to give a brown oil. The oil is subjected to flash chromatography, eluting with a mixture of diethyl ether and pentane (1:4 v/v), to give 3-cyclopentyloxy-4-difluoromethoxybenzaldehyde, in the form of a pale yellow oil (4.7 g). [NMR (CDCl₃): 9.92(s,1H);7.5-7.24(m,3H); 6.34-5.96(t,1H); 4.9(m,1H);2.0-1.6(m,8H)].

### REFERENCE EXAMPLE 35

By proceeding in a manner similar to that described in Reference Example 14, but using as the starting material the appropriate quantity of 3-[exobicyclo(2.2.1)hept-5-en-2-yloxy]-4-methoxy- benzaldehyde, there is prepared 2-(3,5-dichloropyrid-4-yl)-1-[3-{exobicyclo(2.2.1)hept-5-en-2-yloxy}-4-methoxyphenyl]ethanol. [NMR(CDCl₃): 1.2-1.4(m,1H); 1.45-1.6(m,1H);1.65-1.8(m,1H); 1.9(m,1H); 2.1 (bs,1H); 2.9(bs,1H); 3.05(bd,J=14Hz,1H); 3.25(dd,J=5Hz,J=12Hz,1H);3.4-3.5(m, 1H);3.84(s,3H) ;4.28(bm, 1H);5.05(m, 1H); 6.02(m, 1H);6.3(m, 1H);6.8-6.95(m,3H),8.42(s,2H)].

### REFERENCE EXAMPLE 36

By proceeding in a manner similar to that described in Example 14, but using as the starting material the appropriate quantity of 4-difluoromethoxy)-3-isopropoxybenzaldehyde there is prepared 2-(3,5-dichloro-4-pyridyl)-1-(4-difluoromethoxy-3-isopropoxyphenyl)-ethanol, in the form of a white solid, m.p. 125-126°C. [Elemental analysis: C,52.3; H,4.4; N,3.5; Cl,18.1%; calculated: C,52.1; H,4.4; N,3.6; Cl,18.1%].

### REFERENCE EXAMPLE 37

A cold (0°C) solution of 4-hydroxymethyl-3,5-dichloropyridine (3.0 g) and N-bromosuccinimide (6.1 g) in dry dimethylformamide (100 mL) is treated with triphenylphosphine (8.9 g), portionwise, during 5 minutes. The resulting red solution is stirred at 0°C for 45 minutes, and then treated with methanol (5 mL), followed by water (300 mL). The mixture is extracted with diethyl ether (4 x 200 mL), the combined organic washings dried over sodium sulfate, and the solvent removed under reduced pressure. The resulting residue is chromatographed on silica gel, eluting with a mixture of diethyl ether and pentane (1:2 v/v), to give 4-bromomethyl-3,5-dichloropyridine (3 g), in the form of an off-white solid, m.p. 40-44°C.

### REFERENCE EXAMPLE 38

A cold (0°C) solution of 4-formyl-3,5-dichloro-pyridine (3.0 g) in ethanol (50 mL) is treated with sodium borohydride (0.7 g), portionwise, during 5 minutes. The resulting mixture is stirred at 0°C for 10 minutes, and then treated with aqueous hydrochloric acid (5 mL;2 M), followed by basification to pH 7 by treatment with saturated aqueous sodium hydrogen carbonate solution. The mixture is diluted with water (500 mL) and extracted with ethyl acetate (4 x 150 mL). The combined organic washings are dried over magnesium sulfate, and the solvent removed under reduced pressure. The resulting residue is recrystallised from t-butyl methyl ether to give 4-hydroxymethyl-3,5-dichloropyridine (2.0 g), in the form of a white solid, m.p. 87-88°C. The compounds of formula I exhibit useful pharmacological activity and accordingly are incorporated into pharmaceutical compositions and used in the treatment of patients suffering from certain medical disorders. More especially, they are cyclic AMP phosphodiesterase inhibitors, in particular type IV cyclic AMP phosphodiesterase inhibitors. The present invention provides compounds of formula I, and compositions containing compounds of formula I, which are of use in a method for the treatment of a patient suffering from, or subject to, conditions which can be ameliorated by the administration of an inhibitor of cyclic AMP phosphodiesterase. For example, compounds within the present invention are useful as bronchodilators and asthma-prophylactic agents and agents for the inhibition of eosinophil accumulation and of the function of eosinophils, e.g. for the treatment of inflammatory airways disease, especially reversible airway obstruction or asthma, and for the treatment of other diseases and conditions characterized by, or having an etiology involving, morbid eosinophil accumulation. As further examples of conditions which can be ameliorated by the administration of inhibitors of cyclic AMP phosphodiesterase such as compounds of formula I there may be mentioned inflammatory diseases, such as atopic dermatitis, urticaria, allergic rhinitis, psoriasis, rheumatic arthritis, ulcerative colitis, Crohn's disease, adult respiratory distress syndrome and diabetes insipidus, other proliferative skin diseases such as keratosis and various types of dermatitis, conditions associated with cerebral metabolic inhibition, such as cerebral senility, multi-infarct dementia, senile dementia (Alzheimer's disease), and memory impairment associated with Parkinson's disease, and conditions ameliorated by neuroprotectant activity, such as cardiac arrest, stroke, and intermittent claudication. A special embodiment of the therapeutic methods of the present invention is the treating of asthma.

The compounds are also inhibitors of tumor necrosis factor, especially α-TNF. Thus, the present invention provides compounds of formula I, and compositions containing compounds of formula I, which are of use in a method for treating a patient suffering from, or subject to, conditions which can be ameliorated by the administration of an inhibitor of α-TNF. For example compounds of the present invention are useful in joint inflammation, arthritis, rheumatoid arthritis and other arthritic conditions such as rheumatoid spondylitis and osteoarthritis. Additionally, the compounds are useful in treatment of sepsis, septic shock, gram negative sepsis, toxic shock syndrome, acute respiratory distress syndrome, asthma and other chronic pulmonary diseases, bone resorption diseases, reperfusion injury, graft vs. host reaction and allograft rejection. Furthermore, the compounds are useful in the treatment of infections such as viral infections and parasitic infections, for example malaria such as cerebral malaria, fever and myalgias due to infection, HIV, AIDS, cachexia such as cachexia secondary to AIDS or to cancer. Other disease states that may be treated with the compounds of the present invention include Crohn's disease, ulcerative colitis, pyresis, systemic lupus erythematosus, multiple sclerosis, type I diabetes mellitus, psoriasis, Beçhet's disease, anaphylactoid purpura nephritis, chronic glomerulonephritis, inflammatory bowel disease and leukemia. A special embodiment of the therapeutic methods of the present invention is the treating of joint inflammation.

According to a further feature of the invention there is provided a method for the treatment of a human or animal patient suffering from, or subject to, conditions which can be ameliorated by the administration of an inhibitor of cyclic AMP phosphodiesterase or of TNF, especially α-TNF, for example conditions as hereinbefore described, which comprises the administration to the patient of an effective amount of compound of formula I or a composition containing a compound of formula I. "Effective amount" is meant to describe an amount of compound of the present invention effective in inhibiting cyclic AMP phosphodiesterase and/or TNF and thus producing the desired therapeutic effect.

The present invention also includes within its scope pharmaceutical formulations which comprise at least one of the compounds of formula I in association with a pharmaceutically acceptable carrier or coating.

In practice compounds of the present invention may generally be administered parenterally, rectally or orally, but they are preferably administered by inhalation.

The products according to the invention may be presented in forms permitting administration by the most suitable route and the invention also relates to pharmaceutical compositions containing at least one product according to the invention which are suitable for use in human or veterinary medicine. These compositions may be prepared according to the customary methods, using one or more pharmaceutically acceptable adjuvants or excipients. The adjuvants comprise, inter alia, diluents, sterile aqueous media and the various non-toxic organic solvents. The compositions may be presented in the form of tablets, pills, granules, powders, aqueous solutions or suspensions, injectable solutions, elixirs or syrups, and can contain one or more agents chosen from the group comprising sweeteners, flavorings, colorings, or stabilizers in order to obtain pharmaceutically acceptable preparations.

The choice of vehicle and the content of active substance in the vehicle are generally determined in accordance with the solubility and chemical properties of the product, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulfate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

For parenteral administration, emulsions, suspensions or solutions of the products according to the invention in vegetable oil, for example sesame oil, groundnut oil or olive oil, or aqueous-organic solutions such as water and propylene glycol, injectable organic esters such as ethyl oleate, as well as sterile aqueous solutions of the pharmaceutically acceptable salts, are used. The solutions of the salts of the products according to the invention are especially useful for administration by intramuscular or subcutaneous injection. The aqueous solutions, also comprising solutions of the salts in pure distilled water, may be used for intravenous administration with the proviso that their pH is suitably adjusted, that they are judiciously buffered and rendered isotonic with a sufficient quantity of glucose or sodium chloride and that they are sterilized by heating, irradiation or microfiltration.

Suitable compositions containing the compounds of the invention may be prepared by conventional means. For example, compounds of the invention may be dissolved or suspended in a suitable carrier for use in a nebulizer or a suspension or solution aerosol, or may be absorbed or adsorbed onto a suitable solid carrier for use in a dry powder inhaler.

Solid compositions for rectal administration include suppositories formulated in accordance with known methods and containing at least one compound of formula I.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from about 0.001 to about 50, preferably about 0.001 to about 5, mg/kg body weight per day by inhalation, from about 0.01 to about 100, preferably 0.1 to 70, more especially 0.5 to 10, mg/kg body weight per day by oral administration, and from about 0.001 to about 10, preferably 0.01 to 1, mg/kg body weight per day by intravenous administration. In each particular case, the doses will be determined in accordance with the factors distinctive to the subject to be treated, such as age, weight, general state of health and other characteristics which can influence the efficacy of the medicinal product.

The products according to the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day, in accordance with the physiological requirements of each particular patient. Generally, the active product may be administered orally 1 to 4 times per day. It goes without saying that, for other patients, it will be necessary to prescribe not more than one or two doses per day.

Compounds within the scope of the present invention exhibit marked pharmacological activities according to tests described in the literature which tests results are believed to correlate to pharmacological activity in humans and other mammals. The following pharmacological test results are typical characteristics of compounds of the present invention.

### 1. Inhibitory effects of compounds on PDE activity.

### 1.1 Preparation of PDE isozymes from pig aorta.

The method is described fully by Souness and Scott (Biochem. J., 291, 389-395,1993). Briefly, aortas of freshly slaughtered pigs are placed in Hepes buffered krebs solution, extraneous tissue on the outside of the aorta is trimmed off and the endothelial layer on the intimal surface is removed by rubbing with a cotton swab. Smooth muscle strips are plucked from the aorta and 25 g are homogenized using a Waring Blender in homogenization buffer (20 mM Tris/HCl, pH 7.5, 2 mM MgCl₂, 1 mM dithiothreitol, 5 mM EDTA and 1mg/ml aprotinin). The homogenate is further homogenized with an Uttra-Turrax and then centrifuged (3000 g, 5 minutes). The supernatant is removed, and the pellet is sonicated in a small volume (25-50 mL) of homogenization buffer. The sonicate is centrifuged (3000 g, 5 minutes), the pellet discarded and the supernatant is pooled with that from the first centrifugation step. The pooled supernatants are centrifuged (100,000 g, 1 hour), the resulting high-speed supernatant is filtered (0.45 µm) and then applied to a DEAE-trisacryl (IBF) column (50 x 2.44 cm) preequilibrated in column buffer (20 mM Tris/HCl, pH 7.5, 2 mM MgCl₂, 1 mM dithiothreitol, 20 µM TLCK). The column is washed with 500-700 mL of column buffer and PDE activities are eluted with 2 successive linear gradients of NaCl (0-200 mM, 400 mL and 200-300 mM, 200 mL) in column buffer. The fractions in the separated peaks of activity corresponding to the different PDE isozymes are pooled and stored at -20°C in 30% (v/v) ethylene glycol.

### 1.2 Measurement of PDE activity.

PDE activity is determined by the two-step radioisotopic method of Thompson et al., Adv. Cyclic Nucl. Res., 10, 69-92 (1979). The reaction mixture contains 20 mM Tris/HCl (pH 8.0), 10 mM MgCl₂, 4 mM 2-mercaptoethanol, 0.2 mM EGTA and 0.05 mg of BSA/mL. The concentration of substrate is 1 µM.

The IC₅₀ values for the compounds examined are determined from concentration-response curves in which concentrations range from 0.1 nM -to 40 µM.

### 1.3 Results.

Compounds within the scope of the invention produce up to about 50% inhibition of porcine aortic cyclic AMP-specific phosphodiesterase (PDE IV) at concentrations from about 10⁻⁹ M up to about 10⁻⁵ M, preferably from about 10⁻⁹ up to about 10⁻⁸ M. The compounds of the invention are from about 10,000-fold to about 50-fold more selective for cyclic AMP phosphodiesterase IV than cyclic nucleotide phosphodiesterase types I, III or V.

### 2. Inhibitory effects of compounds on eosinophil superoxide generation.

### 2.1 Preparation of guinea-pig eosinophils.

The method is described fully in Souness et al (Biochem. Pharmacol. 42, 937-945, 1991).

### 2.2 Measurement of superoxide generation.

Superoxide anion generation is determined as the superoxide dismutase inhibitable reduction of p-iodonitrotetrazolium violet (INTV) (Souness et al, Biochem. Pharmacol. 42, 937-945, 1991). Briefly, cells are incubated in 96 well microtitre plates in 0.25 mL of Hanks buffered salt solution (HBSS) containing INTV (0.5mg/mL) plus other additions for 45 minutes at 37°C. The cells are then centrifuged at 500 g for 5 minutes and the supernatant is aspirated. The pellet is solubilized by incubation overnight at room temperature in DMSO containing 0.6 M HCl and the absorbance of the reduced dye is measured at 492 nm. The results are expressed in absorbance units.

### 2.3 Results.

Compounds within the scope of the invention produce up to about 50% inhibition of superoxide generation from eosinophiis harvested from the peritoneal cavities of guinea-pigs at concentrations from about 10⁻⁸ M to about 10⁻⁵ M, preferably from about 10⁻⁸ M up to about 10⁻⁷ M.

### 3. Effects of compounds on tracheal smooth muscle contractility.

### 3.1 Preparation of guinea-pig tracheal strips and contractility studies.

Organ bath studies are performed essentially according to Tomkinson et al (Br. J. Pharmacol. 108 57-61, 1993). Briefly, tracheas are removed from male, Dunkin-Hartley guinea-pigs (400-500 g) are placed in Krebs Ringer Bicarbonate (KRB) solution and fat and connective tissue are dissected away. Epithelium is removed by mechanical abrasion and the tracheal strips are suspended under an applied load, such that they are at their optimal length, derived from preliminary experiments, and equilibrated for 90 minutes, washing at 15 minute intervals.

Cumulative concentration-response curves to spasmogens are constructed and the concentration producing 30% of maximum contraction (EC₃₀) is determined by computerized linear regression analysis. For relaxant studies, tissues are contracted with spasmogens (such as methacholine, histamine, leukotriene D₄) (EC₃₀) and when the response plateaus, PDE inhibitors (10 nM-100 µM) or vehicle control (DMSO) are added cumulatively. The concentration of relaxant producing 50% inhibition (IC₅₀) of the agonist response is calculated by linear regression. Altematively, PDE inhibitors, as above, may be added to tissues under basal tone and the concentration producing 50% relaxation (EC₅₀) calculated as above.

### 3.2 Results.

Compounds within the scope of the invention produce about 50% relaxation of guinea-pig tracheal strips (under basal tone or which had been contracted by treatment with spasmogens) at concentrations from about 5x10⁻⁹ M to about 10⁻⁵ M, preferably from about 5x10⁻⁹ M to about 10⁻⁷ M.

### 4. In vivo bronchodilator actions of compounds.

### 4.1 Measurement of bronchodilatation.

Bronchorelaxant activity is measured in in vivo tests in the anaesthetized guinea-pig or rat according to the method described in Underwood et al., Pulm. Pharmacol. 5, 203-212, (1992) in which the effects on bronchospasm induced by histamine (or other spasmogens such as methacholine or leukotriene D₄) is determined. Nebulized aerosols generated from aqueous solutions of compounds of the invention are each administered for one minute to the anaesthetized animals. Alternatively, dry powder formulations made up from compounds of the invention and lactose are blown into the airways of the anaesthetized guinea-pigs or rats by the method described in Underwood et al., J. Pharm. Methods, 26, 203-210, 1991.

### 4.2 Results.

Compounds within the scope of the invention produce from about 30% up to about 90% decrease in bronchospasm when administered at effective doses of about 4 to about 1000 µg/kg, preferably about 4 to about 50 µg/kg, without any significant effect on blood pressure.

### 5 In Vitro Inhibitory Effects on TNF-α Release by Human Monocytes.

The effects of compounds on TNF-α production by human peripheral blood monocytes (PBMs) are examined as follows:

### 5.1. Preparation of blood leukocytes.

Blood is drawn from normal donors, mixed with dextran, and the erythrocytes allowed to sediment for 35 minutes at 37°C. Leukocytes are fractionated by centrifugation through a discontinuous (18, 20 and 22%) metrizamide gradient. The mononuclear cell fraction comprising 30-40% PBMs is suspended in HBSS and stored at 4°C until use.

### 5.2. Measurement of TNFα.

Cells from the PBM-rich metrizamide fraction are spun down (200 g for 10 minutes at 20°C), resuspended at 10⁶ PBMs/mL of medium; RPMI 1640 containing 1%v/v FCS, 50 U/mL penicillin and 50 mg/mL streptomycin (Gibco, U.K.), then plated out in 96 well plates at 2 x10⁵ cells/ well. The medium (200 µL) is changed to remove any non-adherent cells and the remaining, adherent PBMs left in the incubator overnight (18 hours). One hour prior to challenge, the medium is changed to that containing compound for test or drug vehicle. Control treatments and compounds for test are assayed in quadruplicate wells. Compounds are tested within the concentration range of 3 x 10⁻¹⁰ M to 3 x 10⁻⁶ M. Medium (50 µL) with or without 10ng/ml LPS (E. Coli, 055 B5 from Sigma, U.K.) is then added. The incubation is then continued for a further 4 hours. Cell supernatants are removed for storage at -20°C.

TNFα levels in cell supernatants are quantified using a standard sandwich ELISA technique. ELISA plates (Costar, U.K.) are coated overnight at 4°C with 3 mg/mL polyclonal goat anti-human TNFα antibody (British Biotechnology, U.K.) in pH 9.9 bicarbonate buffer. Rabbit polyclonal anti-human TNFα antiserum (Janssen Biochimicha, Belgium) at 1/500 dilution is used as the second antibody and polyclonal goat anti-rabbit IgG horseradish peroxidase (Calbiochem, U.S.A.) at 1/8000 dilution is used as the detection antibody. Color development is measured by absorbance at 450 nm using a Titertek plate reader.

TNF-α levels are calculated by interpolation from a standard curve using recombinant human TNF-α (British Biotechnology U.K.)(0.125-8 ng/mL). Data (log-conc. vs. log-resp) are fitted by linear regression (p > 0.99) using a Multicalc (Wallac Pharmacia, U.K.) software program. Basal TNF-α levels are less than 100 pg/mL whilst LPS stimulation of the PBMs increases TNF-α levels to 3-10 ng/mL.

### 5.3 Results.

Compounds within the scope of the invention produce 50% inhibition of LPS-induced TNF-α release from human PBMs at concentrations within the range of about 10⁻⁹ M to about 10⁻⁶ M., preferably about 10⁻⁹ M to about 10⁻⁸ M.

### 6. In vivo actions of compounds on antigen (ovalbamin)-induced eosinophilia in guinea-pigs.

### 6.1 Treatment of animals and measurement of eosinophil numbers.

Male Dunkin-Hartley guinea-pigs weighing 200-250 g are sensitized using 10 µg ovalbumin in 1 mL of a 100 mg/mL suspension of aluminium hydroxide, i.p.

Sensitized guinea-pigs are anaesthetised and dry powder formulations of PDE inhibitors or lactose are administered (i.t.) into the airways. In some cases PDE inhibitors are administered orally. 23 hours later the procedure is repeated and 60 minutes later the guinea-pigs are challenged with nebulised saline or ovalbumin (1% in saline) for 15 seconds. 24 hours after challenge the guinea-pigs are killed and the lungs are lavaged with warm saline. Total and differential cell counts are made.

### 6.2 Results.

Compounds within the scope of the invention, administered one hour before challenge, inhibit by at least 50% ovalbumin-induced eosinophilia in guinea-pigs which is measured 24 hours after challenge, at oral doses of about 1 to about 50 mg/kg, preferably about 1 to 10 mg/kg and inhaled doses of about 4 to 1000 µg/kg, preferably.4 to 50 µg/kg.

### 7. Inhibitory effects of compounds on antigen-induced bronchoconstriction in the conscious guinea-pig.

### 7.1. Sensitisation of guinea-pigs and measurement of antigen - induced bronchoconstriction.

Male, Dunkin-Hartley guinea-pigs (550-700 g) are sensitized as above. Specific airways resistance (SRaw) is measured in conscious animals by whole body plethysmography using a variation of the method of Pennock et al., (J. Appl. Physiol., 46, 399, 1979). Test compounds or vehicle (lactose carrier) are instilled into the airways as dry powders through a metal gavage needle. 30 minutes later, the animals are injected with mepyramine (30 mg/kg i.p.) to prevent anaphylactic collapse and placed into the plethysmography chambers where SRaw is determined at 1 minute intervals. Resting SRaw is then determined. Animals are challenged with an aerosol of ovalbumin and SRaw is determined every 5 minutes for 15 minutes.

### 7.2. Results.

Compounds within the scope of the invention inhibit antigen-induced bronchoconstriction by up to 80% at doses of between about 1 to about 1000 µg/kg (i.t.), preferably about 1 to about 20 µg/kg (i.t.).

### 8. Inhibitory effects of compounds on serum TNF-α levels in LPS - challenged mice.

### 8.1. Treatment of animals and measurement of murine TNF-α.

Female Balb/c mice (age 6-8 weeks, weight 20-22 g from Charles River, U.K.) in groups of five or more animals are dosed p.o. with compounds suspended in 1.5% (w/v) carboxymethyl cellulose then challenged after a minimum period of 30 min with 30 µg of LPS i.p. After 90 min the animals are killed by CO₂ asphyxiation and bled by cardiac puncture. Blood is allowed to clot at 4°C, centrifuged (12,000 g for 5 minutes) and serum taken for TNF-α analysis.

TNF-α levels are measured using a commercially available murine TNF-α ELISA kit, purchased from Genzyme (Cat. no. 1509.00 ), as recommended by the manufacturer. Values for TNF-α are calculated from a recombinant murine TNF-α standard curve.

### 8.2 Results.

Compounds within the scope of the invention inhibit LPS-induced serum TNF-α at doses between about 10 and about 10,000 µg/kg, preferably about 10 to about 250 µg/kg.

The value of the compounds of the invention is enhanced by their very low mammalian toxicity levels.

The following Composition Examples illustrate pharmaceutical compositions according to the present invention.

### COMPOSITION EXAMPLE 1

3-Cyclopentyloxy-4-methoxyphenyl-2',6'-dichlorobenzyl ketone (1 g) (mean particle size 3.5 microns) and lactose (99 g) (mean particle size 72 microns) are blended together for 30 minutes in a mechanical shaker/mixer. The resulting blend is filled, to a fill weight of 25 mg, into No. 3 hard gelatin capsules, to give a product suitable for use, for example, with a dry powder inhaler.

### COMPOSITION EXAMPLE 2

3-Cyclopentyloxy-4-methoxyphenyl-3,5-dichloropyrid-4-ylmethyl ketone (1 g) (mean particle size 3.5 microns) and lactose (99 g) (mean particle size 72 microns) are blended together for 30 minutes in a mechanical shaker/mixer. The resulting blend is filled, to a fill weight of 25 mg, into No. 3 hard gelatin capsules, to give a product suitable for use, for example, with a dry powder inhaler.

### COMPOSITION EXAMPLE 3

No. 2 size gelatin capsules each containing:-

| | |
|---|---|
| 3-cyclopentyloxy-4-methoxyphenyl-2',6'-dichlorobenzyl ketone | 20 mg |
| lactose | 100 mg |
| starch | 60 mg |
| dextrin | 40 mg |
| magnesium stearate | 1 mg |

are prepared in accordance with the usual procedure.

### COMPOSITION EXAMPLE 4

No. 2 size gelatin capsules each containing:-

| | |
|---|---|
| 3-cyclopentyloxy-4-methoxyphenyl-3,5-dichloropyrid-4-ylmethyl ketone | 20 mg |
| lactose | 100 mg |
| starch | 60 mg |
| dextrin | 40 mg |
| magnesium stearate | 1 mg |

are prepared in accordance with the usual procedure.

### COMPOSITION EXAMPLE 5

3-Cyclopentyloxy-4-methoxyphenyl-2',6'-dichlorobenzyl ketone (1 g) (mean particle size 3.5 microns) and lactose (99 g) (mean particle size 72 microns) are blended together for 30 minutes in a mechanical shaker/mixer. The resulting blend is filled, to a fill weight of 25 mg, into No. 3 hard gelatin capsules, to give a product suitable for use, for example, with a dry powder inhaler.

### COMPOSITION EXAMPLE 6

3-Cyclopentyloxy-4-methoxyphenyl 3,5-dichloropyrid-4-ylmethyl ketone (1 g) (mean particle size 3.5 microns) and lactose (99 g) (mean particle size 72 microns) are blended together for 30 minutes in a mechanical shaker/mixer. The resulting blend is filled, to a fill weight of 25 mg, into No. 3 hard gelatin capsules, to give a product suitable for use, for example, with a dry powder inhaler.

### COMPOSITION EXAMPLE 7

No. 2 size gelatin capsules each containing:-

| | |
|---|---|
| 3-cyclopentyloxy-4-methoxyphenyl-2',6'-dichlorobenzyl ketone | 20 mg |
| lactose | 100 mg |
| starch | 60 mg |
| dextrin | 40 mg |
| magnesium stearate | 1 mg |

are prepared in accordance with the usual procedure.

### COMPOSITION EXAMPLE 8

No. 2 size gelatin capsules each containing:-

| | |
|---|---|
| 3-cyclopentyloxy-4-methoxyphenyl-3,5-dichloropyrid-4-ylmethyl ketone | 20 mg |
| lactose | 100 mg |
| starch | 60 mg |
| dextrin | 40 mg |
| magnesium stearate | 1 mg |

are prepared in accordance with the usual procedure.

## Claims

1. A pharmaceutical composition comprising a compound of formula I 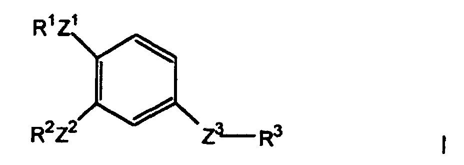 wherein
R¹ is C₁₋₄ alkyl, unsubstituted or substituted by one or more substituents selected from halogen atoms and cycloalkyl or cycloalkenyl groups;
R² is alkyl unsubstituted or substituted by one or more substituents selected from halogen atoms and cycloalkyl or cycloalkenyl groups; alkenyl unsubstituted or substituted by one or more halogen atoms; cycloalkyl unsubstituted or substituted by one or more substituents selected from halogen atoms and methylene (H2C=) or alkyl groups; cycloalkenyl unsubstituted or substituted by one or more halogen atoms; cyclothioalkyl unsubstituted or substituted by one or more halogen atoms; or cyclothioalkenyl unsubstituted or substituted by one or more halogen atoms; each of cyclothioalkyl and cyclothioalkenyl optional oxidized to the corresponding S-oxide or S,S-dioxide;
R³ is aryl or heteroaryl, each of which is unsubstituted or substituted by alkyl, aryl, aralkyl, hydroxy, hydroxyalkyl, alkoxy, aryloxy, aralkoxy, carboxy, acyl, aroyl, halo, nitro, cyano, alkoxycarbonyl, aryloxycarbonyl, aralkoxycarbonyl, acylamino, aroylamino, alkylsulphonyl, arylsulphonyl, alkylsulphinyl, arylsulphinyl, alkylthio, arylthio, aralkylthio, Y¹Y²N-, Y¹Y²NCO- or Y¹Y²NSO₂-, where Y¹ and Y² are independently hydrogen, alkyl, aryl or aralkyl;
Z, Z¹ and Z² are independently oxygen or sulfur;
Z³ is -CH=CH-, -CZCH₂-, -CZ-CZ-, -CH₂-NH-, -NH-CH₂-, -O-CH₂-, -SCH₂-, -SOCH₂-, -SO₂CH₂-, -O-CZ-, -NH-CZ-, -N=N-, or -CZ-CZ-NH-; and
X is halo;
or an N-oxide thereof or a pharmaceutically acceptable salt thereof, together with a pharmaceutically acceptable carrier.

2. A compound of formula I 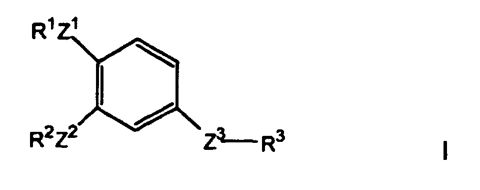 wherein
R¹ is C₁₋₄ alkyl, unsubstituted or substituted by one or more substituents selected from halogen atoms and cycloalkyl or cycloalkenyl groups;
R² is alkyl unsubstituted or substituted by one or more substituents selected from halogen atoms and cycloalkyl or cycloalkenyl groups; alkenyl unsubstituted or substituted by one or more halogen atoms; cycloalkyl unsubstituted or substituted by one or more substituents selected from halogen atoms and methylene (H2C=) or alkyl groups; cycloalkenyl unsubstituted or substituted by one or more halogen atoms; cyclothioalkyl unsubstituted or substituted by one or more halogen atoms; or cyclothioalkenyl unsubstituted or substituted by one or more halogen atoms; each of cyclothioalkyl and cyclothioalkenyl optionally oxidized to the corresponding S-oxide or S,S-dioxide;
R³ is aryl or heteroaryl, each of which is unsubstituted or substituted by alkyl, aryl, aralkyl, hydroxy, hydroxyalkyl, alkoxy, aryloxy, aralkoxy, carboxy, acyl, aroyl, halo, nitro, cyano, alkoxycarbonyl, aryloxycarbonyl, aralkoxycarbonyl, acylamino, aroylamino, alkylsulphonyl, arylsulphonyl, alkylsulphinyl, arylsulphinyl, alkylthio, arylthio, aralkylthio, Y¹Y²N-, Y¹Y²NCO- or Y¹Y²NSO₂-, where Y¹ and Y² are independently hydrogen, alkyl, aryl or aralkyl;
Z, Z¹ and Z² are independently oxygen or sulfur;
Z³ is -CH=CH-, -CZCH₂-, -CZ-CZ-, -CH₂-NH-, -NH-CH₂-, -O-CH₂-, -SCH₂-, -SOCH₂-, -SO₂CH₂-, -O-CZ-, -NH-CZ-, -N=N-, or -CZ-CZ-NH-; and
X is halo;
or an N-oxide thereof or a pharmaceutically acceptable salt thereof, provided that:
when R¹ is methyl, R² is C₃₋₇ alkyl or C₃₋₇ cycloalkyl, Z¹ and Z² are oxygen and R³ is phenyl or phenyl substituted by halo, hydroxy, lower alkoxy, aryloxy, lower alkanoyloxy, amino, lower alkylamino, arylamino or lower alkanoylamino, then Z³ is other than -COCH₂-.

3. The compound or composition according to claim 1 or claim 2 wherein R¹ is C₁₋₄ alkyl substituted by halo.

4. A compound or composition according to claim 1, 2 or 3 wherein R² is cyclopentyl.

5. A compound or composition according to any one of claims 1 to 4 wherein R³ is phenyl substituted in the 2-position or in both the 2- and 6-positions.

6. A compound or composition according to any one of claims 1 to 4 wherein R³ is heteroaryl substituted on one or both of the positions adjacent to the position of R³ that is attached to Z³.

7. A compound or composition according to claim 6 wherein R³ is a 3,5-dihalopyrid-4-yl group or an N-oxide thereof.

8. A compound or composition according to any one of claims 1 to 7 wherein Z¹ and Z² are both oxygen.

9. A compound or composition according to claim 1 or claim 2 in which the compound is selected from:
3-cyclopentyloxy-4-methoxyphenyl-2',6'-dichlorobenryl ketone;
3-cyclopentyloxy-4-methoxyphenyl-3,5-dichloropyrid-4-ylmethyl ketone;
3,5-dichloro-4-(2-(3-cyclopentyloxy-4-methoxyphenyl)-2-oxoethyl)pyridine-N-oxide;
1-(3-cyclopentyloxy-4-methoxyphenyl)-2-(3-chloropyrid-4-yl)ethanone;
1-(3-cyclopentyloxy-4-methoxyphenyl)-2-(4-pyridyl)ethanone;
(3-cyclopentyloxy-4-methoxyphenyl)-2,6-dichlorobenyl ether;
N-(2-chlorophenyl)-3-cyclopentyloxy-4-methoxybenzylamine;
1-(3-cyclopentyloxy-4-methoxyphenyl)-2-(2,6-dichlorophenyl)ethene;
1-(3-cyclopentyloxy-4-methoxyphenyl)-2-(2,6-difluorophenyl)ethene;
1-(3-cyclopentyloxy-4-methoxyphenyl)-2-(pyrid-4-yl)ethane-1,2-dione;
trans-1-(3-cyclopentyloxy-4-methoxyphenyl)-2-(3,5-dichloropyrid-4-yl)diazene;
1-(3-cyclopentyloxy-4-methoxyphenyl)-c-1-oxo-r-2-(3,5-dichloro-1-oxo-pyrid-4-yl)diazene;
trans-1-(3-cyclopentyloxy-4-methoxyphenyl)-2-(3,5-dichloro-1-oxo-pyrid-4-yl)diazene;
(±)-1-[3-{(exo)-8,9,10-trinorbomyl-2-oxy}-4-methoxyphenyl]-2-(3,5-dichloropyrid-4-yl)ethanone;
1-[3-cyclopentyloxy-4-(methylthio)phenyl]-2-(3,5-dichloropyrid-4-yl)ethanone;
1-(4-methoxy-3-prop-2-yloxyphenyl)-2-(3,5-dichforopyrid-4-yl)ethanone;
1-(4-methylthio-3-prop-2-yloxyphenyl)-2-(3,5-dichloropyrid-4-yl)ethanone;
1-(4-methoxy-3-prop-2-yloxyphenyl)-2-(3,5-dichloro-1-oxido-4-pyridinio)ethanone;
1-(3-cyclopentyloxy-4-difluoromethoxyphenyl)-2-(3,5-dichloropyrid-4-yl)ethanone;
1-(3-cyclopentyloxy-4-difluoromethoxyphenyl)-2-(3,5-dichloro-1-oxido-4-pyridinio)ethanone;
2-(3,5-dichloropyrid-4-yl)-1-[3-{exobicyclo(2.2.1)hept-5-en-2-yloxy}-4-methoxyphenyl]ethanone;
2-(3,5-dichloro-4-pyridyl)-1-(4-difluoromethoxy-3-(exo)-8,9,10-trinorbom-2-yloxyphenyl)ethanone;
2-(3,5-dichloro-1-oxido-4-pyridinio)-1-[4-difluoromethoxy-3-(exo)-8,9,10-trinorbom-2-yloxyphenyl]ethanone;
2-(3,5-dichloro-4-pyridyl)-1-[4-methoxy-3-(3-methyl-2-butenyloxy)phenyl]ethanone;
2-(3,5-dichloro-4-pyridyl)-1-(4-difluoromethoxy-3-isopropoxyphenyl)ethanone;
2-(3,5-dichloro-1-oxido-4-pyridinio)-1-(4-difluoromethoxy-3-isopropoxyphenyl)ethanone; or
3,5-dichloro-4-(3-cyclopentyloxy-4-methoxyphenoxymethyl)pyridine.

10. Use of a compound of formula I as defined in claim 1 for the manufacture of a medicament for treating a disease state capable of being modulated by inhibiting cyclic AMP phosphodiesterase.

## Patentansprüche

1. Pnarmazeutische Zusammensetzung, umfassend eine Verbindung der Formel I 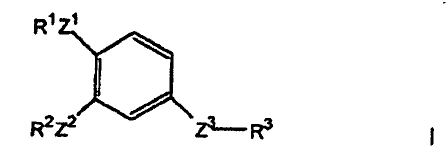 worin
R¹ C₁₋₄-Alkyl, unsubstituiert oder substituiert durch einen oder mehrere Substituenten, ausgewählt aus Halogenatomen und Cycloalkyl- oder Cycloalkenylgruppen, darstellt;
R² Alkyl, unsubstituiert oder substituiert durch einen oder mehrere Substituenten, ausgewählt aus Halogenatomen und Cycloalkyl- oder Cycloalkenylgruppen; Alkenyl, unsubstituiert oder substituiert durch ein oder mehrere Halogenatome; Cycloalkyl, unsubstituiert oder substituiert durch einen oder mehrere Substituenten, ausgewählt aus Halogenatomen und Methylen (H2C=) oder Alkylgruppen; Cycloalkenyl, unsubstituiert oder substituiert durch ein oder mehrere Halogenatome; Cyclothioalkyl, unsubstituiert oder substituiert durch ein oder mehrere Halogenatome; oder Cyclothioalkenyl, unsubstituiert oder substituiert durch ein oder mehrere Halogenatome; jedes Cyclothioalkyl und Cyclothioalkenyl gegebenenfalls zu dem entsprechenden S-Oxid oder S,S-Dioxid oxidiert, darstellt;
R³ Aryl oder Heteroaryl darstellt, wobei jedes unsubstituiert oder substituiert durch Alkyl, Aryl, Aralkyl, Hydroxy, Hydroxyalkyl, Alkoxy, Aryloxy, Aralkoxy, Carboxy, Acyl, Aroyl, Halogen, Nitro, Cyano, Alkoxycarbonyl, Aryloxycarbonyl, Aralkoxycarbonyl, Acylamino, Aroylamino, Alkylsulfonyl, Arylsulfonyl, Alkylsulfinyl, Arylsulfinyl, Alkylthio, Arylthio, Aralkylthio, Y¹Y²N-, Y¹Y²NCO- oder Y¹Y²NSO₂- ist, worin Y¹ und Y² unabhängig voneinander Wasserstoff, Alkyl, Aryl oder Aralkyl darstellen;
Z, Z¹ und Z² unabhängig voneinander Sauerstoff oder Schwefel darstellen;
Z³ -CH=CH-, -CZCH₂-, -CZ-CZ-, -CH₂-NH-, -NH-CH₂-, -O-CH₂-, -SCH₂-, -SOCH₂-, -SO₂CH₂-, -O-CZ-, -NH-CZ-, -N=N- oder -CZ-CZ-NH- darstellt; und
X Halogen darstellt;
oder ein N-Oxid hiervon oder ein pharmazeutisch annehmbares Salz hiervon zusammen mit einem pharmazeutisch annehmbaren Träger.

2. Verbindung der Formel I 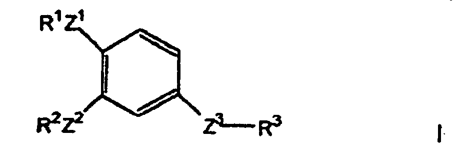 worin
R¹ C₁₋₄-Alkyl, unsubstituiert oder substituiert durch einen oder mehrere Substituenten, ausgewählt aus Halogenatomen und Cycloalkyl- oder Cycloalkenylgruppen, darstellt;
R² Alkyl, unsubstituiert oder substituiert durch einen oder mehrere Substituenten, ausgewählt aus Halogenatomen und Cycloalkyl- oder Cycloalkenylgruppen; Alkenyl, unsubstituiert oder substituiert durch ein oder mehrere Halogenatome; Cycloalkyl, unsubstituiert oder substituiert durch einen oder mehrere Substituenten, ausgewählt aus Halogenatomen und Methylen (H2C=) oder Alkylgruppen; Cycloalkenyl, unsubstituiert oder substituiert durch ein oder mehrere Halogenatome; Cyclothioalkyl, unsubstituiert oder substituiert durch ein oder mehrere Halogenatome; oder Cyclothioalkenyl, unsubstituiert oder substituiert durch ein oder mehrere Halogenatome; jedes Cyclothioalkyl und Cyclothioalkenyl gegebenenfalls zu dem entsprechenden S-Oxid oder S,S-Dioxid oxidiert, darstellt;
R³ Aryl oder Heteroaryl darstellt, wobei jedes unsubstituiert oder substituiert durch Alkyl, Aryl, Aralkyl, Hydroxy, Hydroxyalkyl, Alkoxy, Aryloxy, Aralkoxy, Carboxy, Acyl, Aroyl, Halogen, Nitro, Cyano, Alkoxycarbonyl, Aryloxycarbonyl, Aralkoxycarbonyl, Acylamino, Aroylamino, Alkylsulfonyl, Arylsulfonyl, Alkylsulfinyl, Arylsulfinyl, Alkylthio, Arylthio, Aralkylthio, Y¹Y²N-, Y¹Y²NCO- oder Y¹Y²NSO₂- ist, worin Y¹ und Y² unabhängig voneinander Wasserstoff, Alkyl, Aryl oder Aralkyl darstellen;
Z, Z¹ und Z² unabhängig voneinander Sauerstoff oder Schwefel darstellen;
Z³ -CH=CH-, -CZCH₂-, -CZ-CZ-, -CH₂-NH-, -NH-CH₂-, -O-CH₂-, -SCH₂-, -SOCH₂-, -SO₂CH₂-, -O-CZ-, -NH-CZ-, -N=N- oder-CZ-CZ-NH- darstellt; und
X Halogen darstellt;
oder ein N-Oxid hiervon oder ein pharmazeutisch annehmbares Salz hiervon mit der Maßgabe, dass:
wenn R¹ Methyl darstellt, R² C₃₋₇-Alkyl oder C₃₋₇-Cycloalkyl darstellt, Z¹ und Z² Sauerstoff darstellen und R³ Phenyl oder Phenyl substituiert durch Halogen, Hydroxy, Niederalkoxy, Aryloxy, Niederalkanoyloxy, Amino, Niederalkylamino, Arylamino oder Niederalkanoylamino darstellt, dann Z³ eine andere Bedeutung als -COCH₂- besitzt.

3. Verbindung oder Zusammensetzung gemäß Anspruch 1 oder 2, worin R¹ C₁₋₄-Alkyl, substituiert durch Halogen, darstellt.

4. Verbindung oder Zusammensetzung gemäß Anspruch 1, 2 oder 3, worin R² Cyclopentyl darstellt.

5. Verbindung oder Zusammensetzung gemäß einem der Ansprüche 1 bis 4, worin R³ Phenyl, substituiert in der 2-Stellung oder sowohl in der 2- als auch in der 6-Stellung darstellt.

6. Verbindung oder Zusammensetzung gemäß einem der Ansprüche 1 bis 4, worin R³ Heteroaryl, substituiert an einer oder beiden Stellungen benachbart zu der Stellung von R³, das an Z³ gebunden ist, darstellt.

7. Verbindung oder Zusammensetzung gemäß Anspruch 6, worin R³ eine 3,5-Dihalogenpyrid-4-yl-Gruppe oder ein N-Oxid hiervon darstellt.

8. Verbindung oder Zusammensetzung gemäß einem der Ansprüche 1 bis 7, worin Z¹ und Z² beide Sauerstoff darstellen.

9. Verbindung oder Zusammensetzung gemäß Anspruch 1 oder 2, worin die Verbindung ausgewählt ist aus:
3-Cyclopentyloxy-4-methoxyphenyl-2',6'-dichlorbenzylketon;
3-Cyclopentyloxy-4-methoxyphenyl-3,5-dichlorpyrid-4-yl-methylketon;
3,5-Dichlor-4-(2-(3-cyclopentyloxy-4-methoxyphenyl)-2-oxoethyl)-pyridin-N-oxid;
1-(3-Cyclopentyloxy-4-methoxyphenyl)-2-(3-chlorpyrid-4-yl)-ethanon;
1-(3-Cyclopentyloxy-4-methoxyphenyl)-2-(4-pyridyl)-ethanon;
(3-Cyclopentyloxy-4-methoxyphenyl)-2,6-dichlorbenzylether;
N-(2-Chlorphenyl)-3-cyclopentyloxy-4-methoxybenzylamin;
1-(3-Cyclopentyloxy-4-methoxyphenyl)-2-(2,6-dichlorphenyl)-ethen;
1-(3-Cyclopentyloxy-4-methoxyphenyl)-2-(2,6-difluorphenyl)-ethen;
1-(3-Cyclopentyloxy-4-methoxyphenyl)-2-(pyrid-4-yl)-ethan-1,2-dion;
trans-1-(3-Cyclopentyloxy-4-methoxyphenyl)-2-(3,5-dichlorpyrid-4-yl)-diazen;
1-(3-Cyclopentyloxy-4-methoxyphenyl)-c-1-oxo-r-2-(3,5-dichlor-1-oxo-pyrid-4-yl)-diazen;
trans-1-(3-Cyclopentyloxy-4-methoxyphenyl)-2-(3,5-dichlor-1-oxo-pyrid-4-yl)-diazen;
(±)-1-[3-{(Exo)-8,9,10-trinorbornyl-2-oxy}-4-methoxyphenyl]-2-(3,5-dichlorpyrid-4-yl)-ethanon;
1-[3-Cyclopentyloxy-4-(methylthio)-phenyl]-2-(3,5-dichlorpyrid-4-yl)-ethanon;
1-(4-Methoxy-3-prop-2-yl-oxyphenyl)-2-(3,5-dichlorpyrid-4-yl)-ethanon;
1-(4-Methylthio-3-prop-2-yl-oxyphenyl)-2-(3,5-dichlorpyrid-4-yl)-ethanon;
1-(4-Methoxy-3-prop-2-yl-oxyphenyl)-2-(3,5-dichlor-1-oxido-4-pyridinio)-ethanon;
1-(3-Cyclopentyloxy-4-difluormethoxyphenyl)-2-(3,5-dichlorpyrid-4-yl)-ethanon;
1-(3-Cyclopentyloxy-4-difluormethoxyphenyl)-2-(3,5-dichlor-1-oxido-4-pyridinio)-ethanon;
2-(3,5-Dichlorpyrid-4-yl)-1-[3- {exobicyclo-(2.2.1)-hept-5-en-2-yl-oxy}-4-methoxyphenyl]-ethanon;
2-(3,5-Dichlor-4-pyridyl)-1-(4-difluormethoxy-3-(exo)-8,9,10-trinorborn-2-yl-oxyphenyl)-ethanon;
2-(3,5-Dichlor-1-oxido-4-pyridinio)-1-[4-difluormethoxy-3-(exo)-8,9,10-trinorborn-2-yloxyphenyl]-ethanon;
2-(3,5-Dichlor-4-pyridyl)-1-[4-methoxy-3-(3-methyl-2-butenyloxy)-phenyl]-ethanon;
2-(3,5-Dichlor-4-pyridyl)-1-(4-difluormethoxy-3-isopropoxyphenyl)-ethanon;
2-(3,5-Dichlor-1-oxido-4-pyridinio)-1-(4-difluormethoxy-3-isopropoxyphenyl)-ethanon; oder
3,5-Dichlor-4-(3-cyclopentyloxy-4-methoxyphenoxymethyl)-pyridin.

10. Verwendung einer Verbindung der Formel I wie in Anspruch 1 definiert zur Herstellung eines Arzneimittels zur Behandlung eines Krankheitszustands, der durch Inhibierung cyclischer AMP-Phosphodiesterase einstellbar ist.

## Revendications

1. Composition pharmaceutique comprenant un composé de formule I 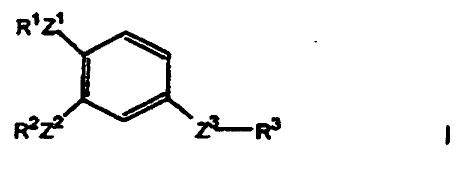 dans laquelle
R¹ est un alkyle en C₁₋₄, non substitué ou substitué par un ou plusieurs substituants sélectionnés parmi des atomes d'halogène et des groupements cycloalkyle ou cycloalcényle;
R² est un alkyle non substitué ou substitué par un ou plusieurs substituants sélectionnés parmi des atomes d'halogène et des groupements cycloalkyle ou cycloalcényle; un alcényle non substitué ou substitué par un ou plusieurs atomes d'halogène; un cycloalkyle non substitué ou substitué par un ou plusieurs substituants sélectionnés parmi des atomes d'halogène et des groupements méthylène (H₂C=) ou alkyle; un cycloalcényle non substitué ou substitué par un ou plusieurs atomes d'halogène; un cyclothioalkyle non substitué ou substitué par un ou plusieurs atomes d'halogène, ou un cyclothioalcényle non substitué ou substitué par un ou plusieurs atomes d'halogène, chacun des cyclothioalkyle et cyclothioalcényle éventuellement oxydé en le S-oxyde ou le S,S-dioxyde correspondant;
R³ est un aryle ou un hétéroaryle, dont chacun est non substitué ou substitué par un alkyle, un aryle, un arylalkyle, un hydroxy, un hydroxyalkyle, un alkoxy, un aryloxy, un arylalkoxy, un carboxy, un acyle, un aroyle, un halogéno, un nitro, un cyano, un alkoxycarbonyle, un aryloxycarbonyle, un arylalkoxycarbonyle, un acylamino, un aroylamino, un alkylsulfonyle, un arylsulfonyle, un alkylsulfinyle, un arylsulfinyle, un alkylthio, un arylthio, un arylalkylthio, Y¹Y²N-, Y¹Y²NCO- ou Y¹Y²NSO₂-, dans lesquels Y¹ et Y² sont indépendamment hydrogène, alkyle, aryle ou arylalkyle;
Z, Z¹ et Z² sont indépendamment oxygène ou soufre;
Z³ est -CH=CH-, -CZCH₂-, -CZ-CZ-, -CH₂-NH-, -NH-CH₂-, -O-CH₂-, -SCH₂-, -SOCH₂-, -SO₂CH₂-, -O-CZ-, -NH-CZ-, -N=N- ou -CZ-CZ-NH-, et
X est un halogéno,
ou un de ses N-oxydes ou un de ses sels pharmaceutiquement acceptables, ensemble avec un vecteur pharmaceutiquement acceptable.

2. Composé de formule I 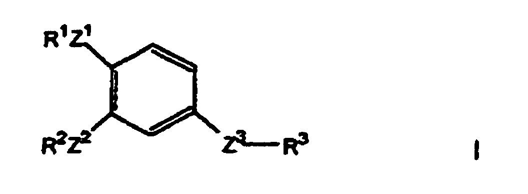 dans laquelle
R¹ est un alkyle en C₁₋₄, non substitué ou substitué par un ou plusieurs substituants sélectionnés parmi des atomes d'halogène et des groupements cycloalkyle ou cycloalcényle;
R² est un alkyle non substitué ou substitué par un ou plusieurs substituants sélectionnés parmi des atomes d'halogène et des groupements cycloalkyle ou cycloalcényle; un alcényle non substitué ou substitué par un ou plusieurs atomes d'halogène; un cycloalkyle non substitué ou substitué par un ou plusieurs substituants sélectionnés parmi des atomes d'halogène et des groupements méthylène (H₂C=) ou alkyle; un cycloalcényle non substitué ou substitué par un ou plusieurs atomes d'halogène; un cyclothioalkyle non substitué ou substitué par un ou plusieurs atomes d'halogène, ou un cyclothioalcényle non substitué ou substitué par un ou plusieurs atomes d'halogène, chacun des cyclothioalkyle et cyclothioalcényle éventuellement oxydé en le S-oxyde ou le S,S-dioxyde correspondant;
R³ est un aryle ou un hétéroaryle, dont chacun est non substitué ou substitué par un alkyle, un aryle, un arylalkyle, un hydroxy, un hydroxyalkyle, un alkoxy, un aryloxy, un arylalkoxy, un carboxy, un acyle, un aroyle, un halogéno, un nitro, un cyano, un alkoxycarbonyle, un aryloxycarbonyle, un arylalkoxycarbonyle, un acylamino, un aroylamino, un alkylsulfonyle, un arylsulfonyle, un alkylsulfinyle, un arylsulfinyle, un alkylthio, un arylthio, un arylalkylthio, Y¹Y²N-, Y¹Y²NCO- ou Y¹Y²NSO₂-, dans lesquels Y¹ et Y² sont indépendamment hydrogène, alkyle, aryle ou arylalkyle;
Z, Z¹ et Z² sont indépendamment oxygène ou soufre;
Z³ est -CH=CH-, -CZCH₂-, -CZ-CZ-, -CH₂-NH-, -NH-CH₂-, -O-CH₂-, -SCH₂-, -SOCH2-, -SO₂CH₂-, -O-CZ-, -NH-CZ-, -N=N- ou -CZ-CZ-NH-, et
X est un halogéno,
étant entendu que:
lorsque R¹ est un méthyle, R² est un alkyle en C₃₋₇ ou un cycloalkyle en C₃₋₇, Z¹ et Z² sont des oxygènes et R³ est un phényle ou un phényle substitué par un halogéno, un hydroxy, un alkoxy inférieur, un aryloxy, un alcanoyloxy inférieur, un amino, un alkylamino inférieur, un arylamino ou un alcanoylamino inférieur, alors Z³ est différent de -COCH₂-.

3. Composé ou composition suivant la revendication 1 ou 2 dans lequel R¹ est un alkyle en C₁₋₄ substitué par un halogéno.

4. Composé ou composition suivant la revendication 1, 2 ou 3 dans lequel R² est un cyclopentyle.

5. Composé ou composition suivant l'une quelconque des revendications 1 à 4 dans lequel R³ est un phényle substitué en position 2 ou à la fois en les positions 2 et 6.

6. Composé ou composition suivant l'une quelconque des revendications 1 à 4 dans lequel R³ est un hétéroaryle substitué en une des ou en les deux positions adjacentes à la position de R³ qui est liée à Z³.

7. Composé ou composition suivant la revendication 6 dans lequel R³ est un groupement 3,5-dihalogénopyrid-4-yle ou un de ses N-oxydes.

8. Composé ou composition suivant l'une quelconque des revendications 1 à 7 dans lequel Z¹ et Z² sont tous deux des oxygènes.

9. Composé ou composition suivant la revendication 1 ou 2 dans lequel le composé est sélectionné parmi:
la 3-cyclopentyloxy-4-méthoxyphényl-2',6'-dichlorobenzylcétone;
la 3-cyclopentyloxy-4-méthoxyphényl-3,5-dichloropyrid-4-ylméthylcétone;
le N-oxyde de la 3,5-dichloro-4-(2-(3-cyclopentyloxy-4-méthoxyphényl)-2-oxoéthyl)pyridine;
la 1-(3-cyclopentyloxy-4-méthoxyphényl)-2-(3-chloropyrid-4-yl)éthanone;
la 1-(3-cyclopentyloxy-4-méthoxyphényl)-2-(4-pyridyl)éthanone;
l'éther (3-cyclopentyloxy-4-méthoxyphényl)-2,6-dichlorobenzylique,
la N-(2-chlorophényl)-3-cyclopentyloxy-4-méthoxybenzylamine;
le 1-(3-cyclopentyloxy-4-méthoxyphényl)-2-(2,6-dichlorophényl)éthène;
le 1-(3-cyclopentyloxy-4-méthoxyphényl)-2-(2,6-difluorophényl)éthène;
la 1-(3-cyclopentyloxy-4-méthoxyphényl)-2-(pyrid-4-yl)éthane-1,2-dione;
le trans-1-(3-cyclopentyloxy-4-méthoxyphényl)-2-(3,5-dichloropyrid-4-yl)diazène;
le 1-(3-cyclopentyloxy-4-méthoxyphényl)-c-1-oxo-r-2-(3,5-dichloro-1-oxo-pyrid-4-yl)diazène;
le trans-1-(3-cyclopentyloxy-4-méthoxyphényl)-2-(3,5-dichloro-1-oxo-pyrid-4-yl)diazène;
la (±)-1-[3-{(exo)-8,9,10-trinorbornyl-2-oxy)-4-méthoxyphényl]-2-(3,5-dichloropyrid-4-yl)éthanone;
la 1-[3-cyclopentyloxy-4-(méthylthio)phényl]-2-(3,5-dichloropyrid-4-yl)éthanone;
la 1-(4-méthoxy-3-prop-2-yloxyphényl)-2-(3,5-dichloropyrid-4-yl)éthanone;
la 1-(4-méthylthio-3-prop-2-yloxyphényl)-2-(3,5-dichloropyrid-4-yl)éthanone;
la 1-(4-méthoxy-3-prop-2-yloxyphényl)-2-(3,5-dichloro-1-oxido-4-pyridinio)éthanone;
la 1-(3-cyclopentyloxy-4-difluorométhoxyphényl)-2-(3,5-dichloropyrid-4-yl)éthanone;
la 1-(3-cyclopentyloxy-4-difluorométhoxyphényl)-2-(3,5-dichloro-1-oxido-4-pyridinio)éthanone;
la 2-(3,5-dichloropyrid-4-yl)-1-[3-{exobicyclo-(2.2.1)hept-5-èn-2-yloxy)-4-méthoxyphényl)-éthanone;
la 2-(3,5-dichloro-4-pyridyl)-1-(4-difluorométhoxy-3-(exo)-8,9,10-trinorborn-2-yloxyphényl)-éthanone;
la 2-(3,5-dichloro-1-oxido-4-pyridinio)-1-[4-difluorométhoxy-3-(exo)-8,9,10-trinorborn-2-yloxyphényl]éthanone;
la 2-(3,5-dichloro-4-pyridyl)-1-[4-méthoxy-3-(3-méthyl-2-butényloxy)phényl]éthanone;
la 2-(3,5-dichloro-4-pyridyl)-1-(4-difluorométhoxy-3-isopropoxyphényl)éthanone;
la 2-(3,5-dichloro-1-oxido-4-pyridinio)-1-(4-difluorométhoxy-3-isopropoxyphényl)éthanone, ou
la 3,5-dichloro-4-(3-cyclopentyloxy-4-méthoxyphénoxyméthyl)pyridine.

10. Utilisation d'un composé de formule I suivant la revendication 1 pour la fabrication d'un médicament destiné à traiter un état pathologique pouvant être modulé par inhibition de l'AMP cyclique phosphodiestérase.
